# EUROPEAN PATENT SPECIFICATION

(11) **EP 1 642 982 B1**
(45) Date of publication and mention of the grant of the patent: **22.05.2013**
(21) Application number: 04742039.3
(22) Date of filing: 02.07.2004
(51) Int. Cl.: C12N 15/86, C12N 15/863

(54) **RECOMBINANT DNA VIRUSES COMPRISING A DNA THAT IS COMPLEMENTARY TO AN RNA VIRUS REPLICON, AND APPLICATIONS THEREOF**
REKOMBINANTE DNA-VIREN MIT EINER ZU EINEM RNA-VIRUS-REPLIKON KOMPLEMENTÄREN DNA UND ANWENDUNGEN DAVON
RECOMBINANTS DU VIRUS DE L'ADN COMPRENANT UN ADN COMPLEMENTAIRE D'UN REPLICON D'UN VIRUS ARN ET LEURS APPLICATIONS

(30) Priority: 02.07.2003 ES 200301545
(43) Date of publication of application: 05.04.2006
(73) Proprietor: Instituto Nacional de Investigación y Tecnología Agraria y Alimentaria (INIA), 28040 Madrid (ES)
(72) Inventor: SANCHEZ PUIG, Juana Maria, E-28040 Madrid (ES); LORENZO GILSANZ, Maria del Mar, E-28040 Madrid (ES); BLASCO LOZANO, Rafael, E-28040 Madrid (ES)
(74) Representative: ABG Patentes, S.L.
(86) International application number: PCT/ES2004/000310
(87) International publication number: WO 2005/003363

(56) References cited:
- WO-A2-02/18585
- KONETSCHNY C. ET AL.: 'Generation of transduction-competent retroviral vectors by infection with a single hybrid vaccina virus' JOURNAL OF VIROLOGY vol. 77, no. 12, June 2003, pages 7017 - 7025, XP002994681
- LILJEQVIST S. ET AL.: 'Production of recombinant subunit vaccines: protein immunogens, live delivery systems and nucleic acid vaccines' JOURNAL OF BIOTECHNOLOGY vol. 73, 1999, pages 1 - 33, XP004180163
- LUNDSTROM K.: 'Latest development in viral vectors for gene therapy' TRENDS IN BIOTECHNOLOGY vol. 21, no. 3, March 2003, pages 117 - 122, XP004412608
- VAN RIJN P.A. ET AL.: 'An experimental marker vaccine and accompanying serological diagnostic test both based on envelope glycoprotein E2 of classical swine fever virus (CSFV)' VACCINE vol. 17, 1999, pages 433 - 440, XP004153825

## Description

The invention relates, in general, to DNA recombinant viruses whose genome comprises a complementary DNA sequence of a replicon deriving from an RNA virus that contains a sequence of nucleotides that encode an exogenous protein, or fragment thereof, and a sequence of nucleotides that encode structural or encapsidating proteins of said RNA virus. Said recombinant DNA viruses are useful for producing single-cycle chimeric virus particles containing a replicon deriving from an RNA virus, to produce.exogenous proteins of interest, or fragments thereof, and also for therapeutic applications.

### BACKGROUND OF THE INVENTION

Advances in recombinant DNA technology have led to progress in the development of gene transfer between organisms. Many efforts are currently being made to try and produce chemical, pharmaceutical and biological products of economic and commercial interest by using gene transfer techniques.

Immunisation using recombinant viral vectors is a powerful tool for the development of vaccines. It is based on the inoculation of expression vectors that contain nucleotide sequences that encode the immunogenic protein (the exogenous protein of interest). However, the doses that are usually used in a great many studies of vaccinations with poxviruses or other viral vectors are very high, which makes it questionable whether this is viable on a very large scale.

Also, replicons, or (isolated) self-replicating RNA molecules, deriving from single-strand positive-sense RNA viruses, as an alternative to DNA for genetic immunisation. These replicons can also express an exogenous protein of interest. However, this alternative presents many problems due to the high production costs, the low efficiency of the methods for inserting the replicon into the cells, the short intracellular half-life of RNA and its degradation by RNAases. To avoid these problems, systems have been developed to allow the packaging of replicons, generating recombinant virus particles that act as a vehicle for the insertion of the replicons into the cells. However, the production of said particles is difficult and expensive, and the doses used in many studies of vaccinations with alphaviruses are also very high, which makes it questionable whether this is viable on a very large scale.

The usual methods of generating packaged replicons are based on the in vitro transcription of the replicon and subsequent electroporation. Recently, other methods have been developed that are based on the use of packaging cell lines that enable the packaging of replicons. Patent application WO 02/077221 describes packaging cells that produce alphaviruses. However, these methods of generating packaged replicons are expensive and not easily scalable to a an industrial scale.

Furthermore, the use of vaccine vectors occasionally presents intrinsic safety problems, for example, in immunodepressed individuals. This problem can be solved by using single-cycle viral vectors; however, this solution presents the drawback that, with the vaccine virus not being amplified, the amount of antigen would be limited.

Therefore, it is necessary to develop alternative systems of expression of exogenous products of interest, including products with therapeutic effects that can solve all or part of the aforementioned drawbacks.

The invention is illustrated through the construction of a recombinant vaccine virus that comprises a replicon of the Semliki Forest virus which expresses a reporter protein. The Semliki Forest virus (SFV) belongs to the alphavirus family and is a single-strand, positive-sense enveloped RNA virus (Schlesinger and Garoff 1987). The RNA molecule is *capped* and polyadenylated and its approximate size is 11 to 12 kpb (Strauss and Strauss 1994).

By identifying the elements that act in cis for RNA replication and for packaging, it is possible to design expression vectors based on alphaviruses that can express any gene of interest in the cytoplasm of any permissive cell for replication of the virus (Berglund, Sjoberg et al. 1993), (Liljestrom and Garoff 1992). The vectors deriving from these viruses are widely used for in vitro gene expression studies and are beginning to be used for the development of vaccines and applications in gene therapy (Schlesinger and Dubensky 1999). Alphaviruses offer several advantages such as their wide host range (including mammal, insect and bird cells), high levels of protein expression and a small genome that is easy to handle.

After the virus has entered the cell, the genomic RNA acts as an RNA messenger for the translation of the non-structural viral proteins needed to initiate the amplification of viral RNA. The replication of viral RNA begins with the synthesis of a negative-sense intermediary strand that is used as a template for the synthesis of genomic RNA and for the transcription, from an internal promoter, of positive-strand subgenomic RNA. This subgenomic RNA is the RNA messenger for the translation of structural proteins. The synthesis of positive and negative-sense and subgenomic RNA is temporarily regulated by proteolytic processing of non-structural components of the replicase polyprotein, by a protease encoded by the virus in the C-terminal region of the nsP2 non-structural protein (Lemm, Rumenapf et al. 1994).

Expression systems based on alphaviruses have been developed by substituting the genes that they encode for the structural proteins for the gene that is to be expressed. This system has been described for the Sindbis (Bredenbeek, Frolov et al. 1993) and Semliki Forest (Berglund, Sjoberg et al. 1993), (Liljestrom and Garoff 1991) viruses. RNA transcribed in vitro from the resulting construction can be inserted into cells by electroporation (Liljestrom and Garoff 1991) or by a treatment with lipofectin (Xiong, Levis et al. 1989). This RNA is self-replicating because it encodes for the viral replicase, and it also produces high levels of subgenomic RNA that encodes for the protein that is to be expressed (Bredenbeek and Rice 1992). Expression systems based on vaccinia viruses have been developed as well (Konetschny, Holzer et al. 2003) (WO 02/018585). One of these systems is based on recombinant vaccinia viruses that express components of a retroviral vector (*gag-pol* gene, *env* gene, and a retroviral vector unit) as separate transcription units under the control of the vaccinia virus promoter (Konetschny, Holzer et al. 2003). Another expression system based on a vaccinia virus (a modified vaccinia virus Ankara or MVA) comprises a cDNA sequence of a replicon derived from a positive-strand RNA virus (psRNAV) under the control of a T7 promoter and a foreign gene (WO 02/018585). Vaccinia virus-based virus production systems have been described (Holzer, Mayrhofer at al. 1999), comprising a modified retroviral transcription unit under the control of the vaccinia virus early promoter.

In the expression system based on SFV, the production of recombinant particles is based on co-transfection in cells of two independent RNA molecules. The first of these is the replicon, which contains the replicase gene, the subgenomic promoter followed by a heterologic gene that is to be expressed and the 5' and 3' ends of the genome that are needed for RNA replication (Niesters and Strauss 1990), (Kuhn, Hong et al. 1990). The second molecule is the RNA Helper that also conserves the replication signals in 5' and 3' and a subgenomic promoter followed by the genes that encode for the structural proteins (Liljestrom and Garoff 1991) (Liljestrom 1994). Both RNAs can be synthesised in vitro from plasmids that contain those sequences under the SP6 promoter or they can be expressed within the cell by transcription from a eukaryotic promoter (DiCiommo and Bremner 1998). The idea of having the function of Helper encoded by an independent molecule is to achieve, through complementation in trans, a selective recombinant RNA packaging within the SFV particles. This results from the fact that the Helper vector does not have the packaging signal in its sequence. The packaging sign, which is present in the replicon, is recognised by the capsid protein as the virus is being assembled so that in the transfection with the replicon the RNA Helper only produces virus particles that carry the replicon, which means that only the product of the exogenous gene can be expressed in cells that are subsequently infected with particles produced in this manner (Liljestrom and Garoff 1991).

### SUMMARY OF THE INVENTION

A recombinant DNA virus has been developed that comprises all or part of the genome of a DNA virus and a complementary DNA sequence (cDNA) of a replicon deriving from an RNA virus that contains a sequence of nucleotides that encode an exogenous protein, or a fragment thereof, and a sequence of nucleotides that encode encapsidating proteins for said RNA. The recombinant DNA virus acts as a shuttle for the replicon of the RNA virus that contains the encoding sequence of nucleotides of the exogenous protein or fragment thereof. Both the replication of the replicon within the infected cell and the production of particles containing the packaged replicon can act by increasing the amount of exogenous protein, or fragment thereof, produced.

The recombinant DNA virus developed in this invention can be used in basic or applied research, for example, to obtain products of interest, such as vaccine vector or in gene therapy, and also to produce defective single-cycle chimeric virus particles.

A recombinant DNA virus such as that provided by this invention has, among others, numerous advantages in terms of its scalability to mass production of packaged replicons and its use in the production of recombinant vaccines as it makes it possible to amplify the amount of antigen to a greater extent and to use smaller amounts of viral vector. Also, the antigen expression in two different contexts (cells infected by the DNA virus and cells infected with single-cycle particles) can have implications in terms of the immune response against the antigen.

Therefore, in an aspect the invention relates to a recombinant DNA virus that comprises the particulars defined in claim 1.

Another aspect of the invention relates to a procedure for producing defective single-cycle chimeric virus particles deriving from an RNA virus that involves infecting a competent cell with said recombinant DNA viruses.

Another aspect of the invention relates to a pharmaceutical composition that comprises said recombinant DNA viruses and an acceptable pharmaceutical vehicle.

Another aspect of the invention relates to a method for producing a protein of interest, or fragment thereof, that involves infecting a competent cell with said recombinant DNA virus and, if desired, isolating said protein of interest or fragment thereof.

### BRIEF DESCRIPTION OF THE FIGURES

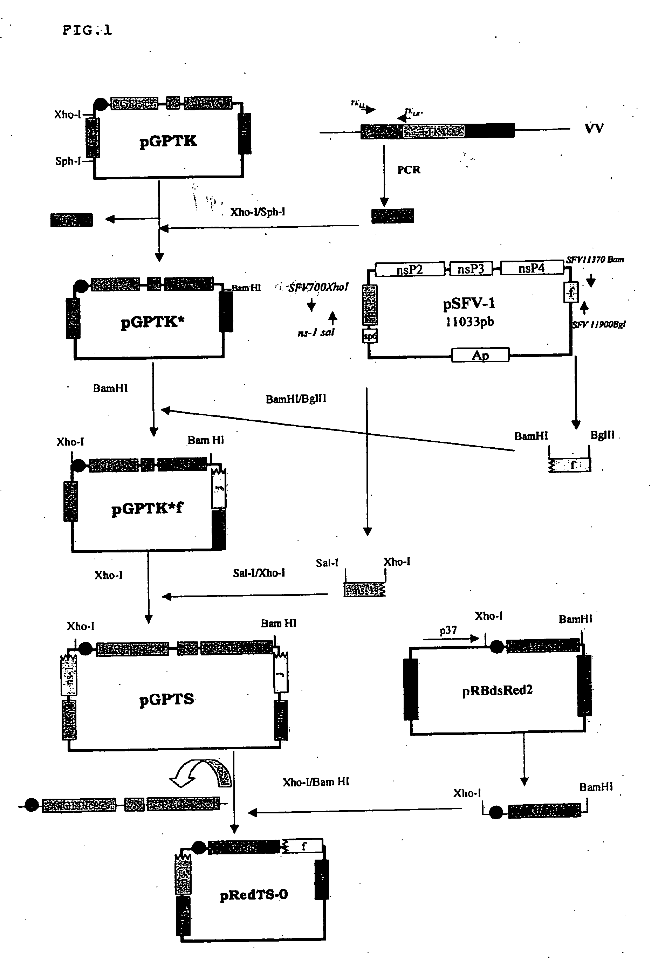

Figure 1 shows a diagram of the construction of the pRedTS-0 plasmid. Part of the 5' and 3' end of the SFV genome was cloned from the pGPTK plasmid, which contains in its sequence the recombination flanks for the thymidine kinase (TK) gene of the vaccinia virus (VV), which subsequently serve as recombination flanks for the full replicon. In the next step the marker gene dsRed is cloned under the control of a viral promoter. TK_{L}: left recombination flank of the TK gene of VV. TK_{R}: right recombination flank of the TK gene of VV. DsRed: red fluorescent protein. ns-1: non-structural part of the SFV gene sequence 1. f: 3' end of the SFV replicon.

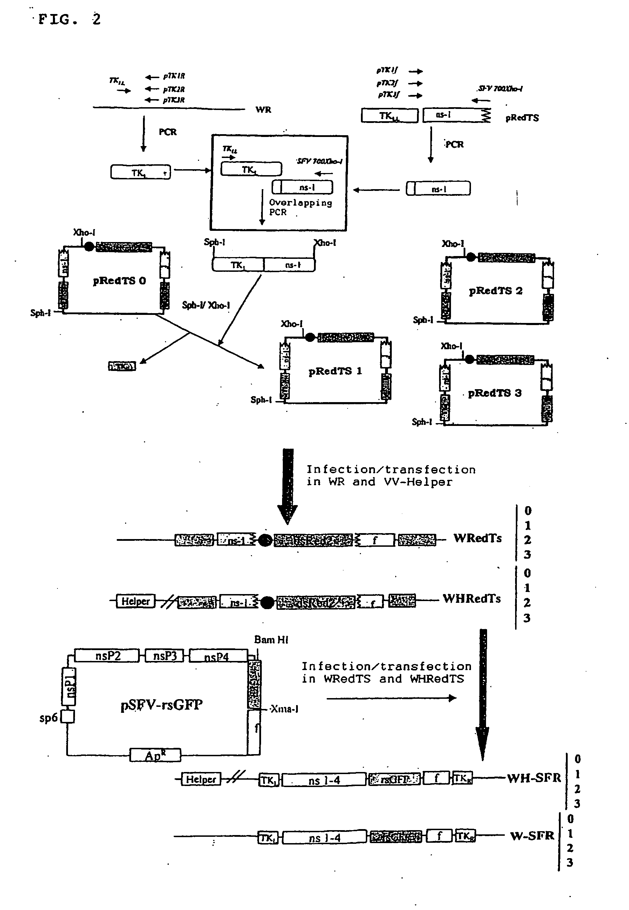

Figure 2 shows a diagram of the construction of pRedTS plasmids 1-3. Mutations were inserted into the natural promoter of TK by recombinant PCR. The 3 versions of the promoter were cloned in the pRedTS-0 plasmid by digestion using Sph-I and Xho-I and three plasmids were generated (pRedTS 1,2 and 3), which are of greater to lesser intensity of expression, respectively. These plasmids were used to generate the viruses WRedTS and WH-RedTS which in turn were used, by transfection of the pSFVrsGFP plasmid, to form the recombinant vaccinia viruses that express the replicon using different promoters (W-SFR-O, W-SFR-1, W-SFR-2, W-SFR-3, WH-SFR-0, WH-SFR-1, WH-SFR-2 and WH-SFR-3).

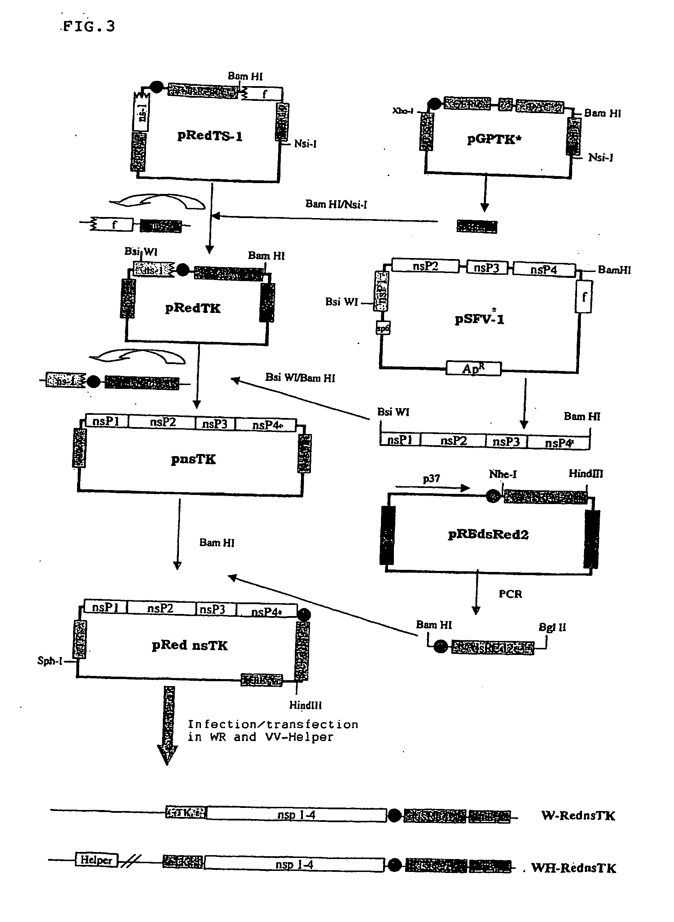

Figure 3 shows a diagram of the construction of the pRednsTK plasmid. By taking the pRedTS plasmid and inserting and removing genes, the pRednsTK plasmid was created, which carries in its sequence the genes of non-structural SFV proteins and the dsRed marker gene. TK_{L}: left recombination flank of the TK gene of VV. TK_{R}: right recombination flank of the TK gene of VV. DsRed: red fluorescent protein. nsP1-4: non-structural SFV genes.

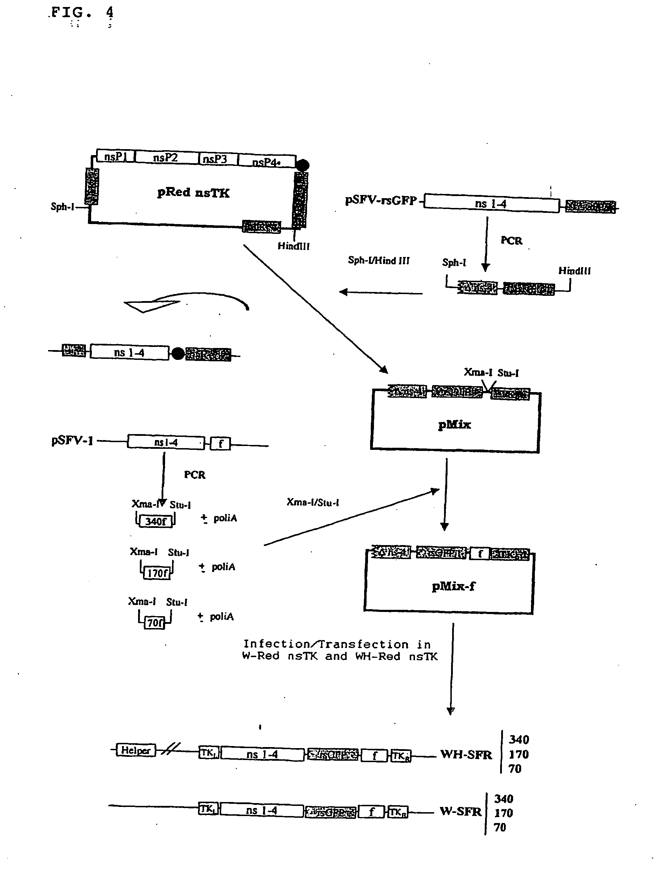

Figure 4 shows a diagram of the construction of the pMix-f plasmid. By taking the pRednsTK plasmid and removing the genes of the structural proteins and the dsRed marker gene and cloning rsGFP and the end part of the nsP4, the pMix plasmid was created. This plasmid was used to clone the three fragments of 340, 170 and 70 nucleotides of the 3' end of the SFV replicon, either including the Poly-A sequence of the SFV cDNA or not, thus generating different versions deriving from the pMixf plasmid (pMix-f-340, pMix-f-340An pMix-f-170, pMix-f-170An, pMix-f-70, pMix-f-70An). ns4: part of the nsP4 gene. rsGFP: green fluorescent protein. f: 3' end of the SFV replicon. TK_{R}: right recombination flank of TK of VV.

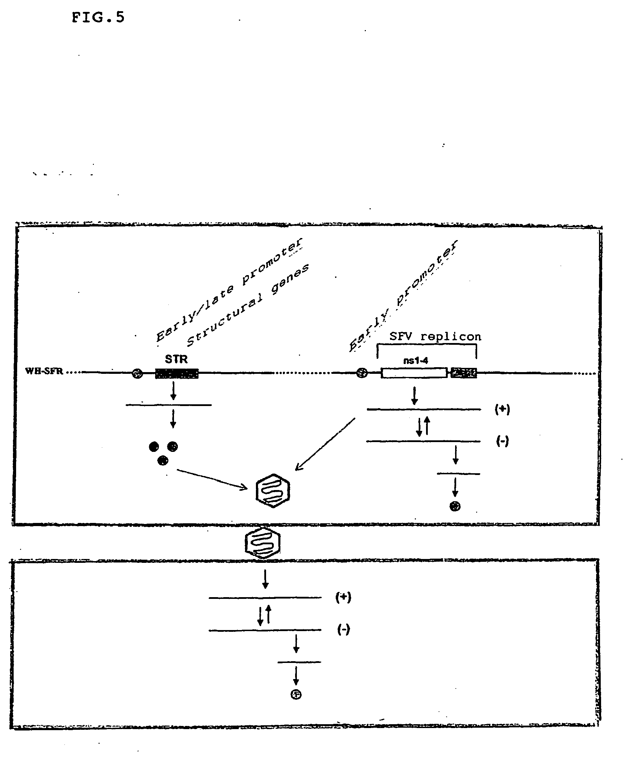

Figure 5 shows a diagram of the design of the combined vaccinia virus-Semliki Forest virus (VV-SFV). In the context of infection with the recombinant vaccinia virus, the structural proteins (STR)and the SFV replicon (nsP1-4 and the gene concerned) are transcribed and translated. The transcription of the replicon from an early promoter of the vaccinia virus gives rise to the positive strand of the replicon (+) which is translated to give rise to replicase. The replicative cycle of the replicon is completed by the negative strand intermediaries (-). Also, a subgenomic messenger is produced that mediates the expression of the exogenous protein (•). The complete replicon (positive strand) together with the structural proteins (STR) expressed from a strong promoter of vaccinia form the single-cycle SFV particles that will infect the surrounding cells and express the protein cloned in the replicon.

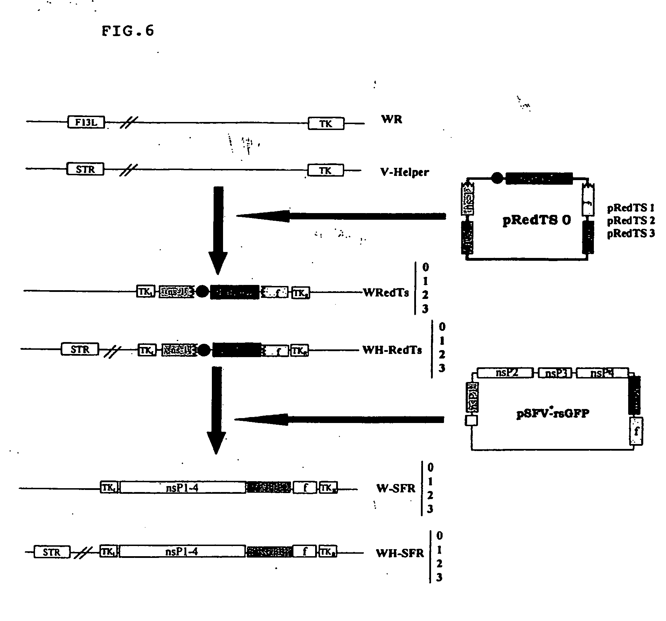

Figure 6 shows a diagram of the construction of recombinant viruses to study the promoter of the vaccinia virus for the expression of the SFV replicon. The W-RedTS and WH-RedTS viruses were isolated from vaccinia virus WR and V-Helper, respectively, by insertion using the pRedTS 0, 1, 2, and 3 plasmids. The V-Helper virus is a recombinant vaccinia virus that expresses the structural proteins of SFV cloned in the F13L locus, under the control of a synthetic early/late promoter, and its isolation is described in the doctoral thesis by Maria del Mar Lorenzo Gilsanz, Faculty of Science, Autonomous University of Madrid, 2001. Subsequently, the viruses shown at the bottom of the figure were isolated by insertion using the pSFVrsGFP plasmid. F13L: gene that encodes the p37 protein. TK: Thymidine kinase gene. STR: Genes of the structural proteins. TK_{L}: left recombination flank of TK. ns-1: 5' end of the replicon dsRed: marker gene. f: 3' end of the replicon. TK_{R}: right recombination flank of TK. nsP1-4: genes of the non-structural proteins. rsGFP: gene of the green fluorescent protein.

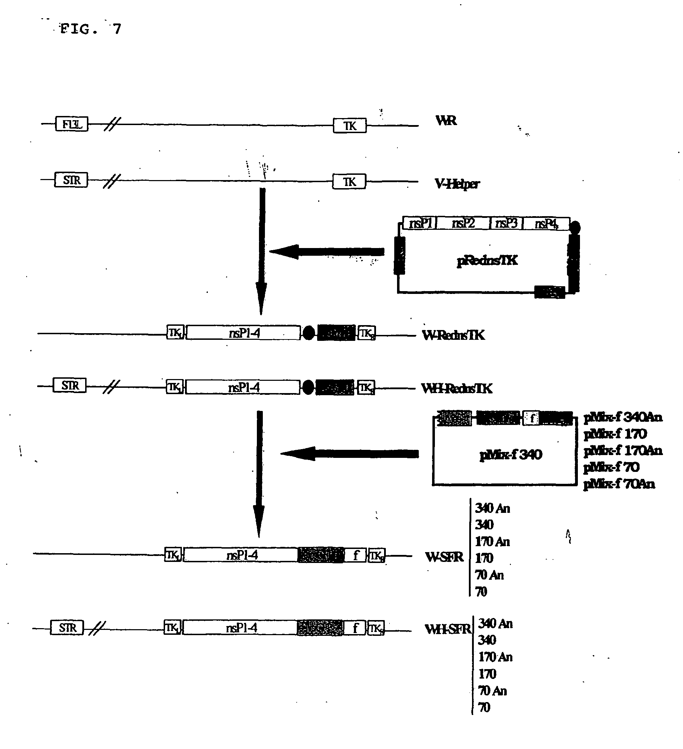

Figure 7 shows a diagram of the construction of recombinant viruses to study the necessary sequence at the 3' end of the replicon. The W-RednsTK and WH-RednsTK viruses were isolated from vaccinia virus WR and V-Helper, respectively, by insertion using the pRednsTK plasmid. Subsequently, the viruses shown at the bottom of the figure were isolated by insertion using the different versions of pMixf plasmids. F13L: gene that encodes the p37 protein. TK: Thymidine kinase gene. STR: Genes of the structural proteins of SFV. TK_{L}: left recombination flank of TK. ns-1: 5' end of the replicon dsRed: marker gene. f: 3' end of the replicon. TK_{R}: right recombination flank of TK. nsP1-4: genes of the non-structural proteins. rsGFP: marker gene.

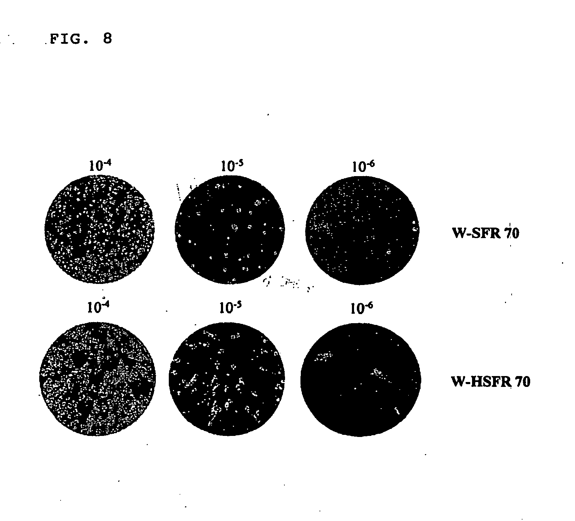

Figure 8 is a set of photographs that show the plaque morphology of the W-SFR 70An and WH-SFR 70An viruses. BSC-1 cells were infected, seeded on 6-well plates, with 1:10 dilutions of the W-SFR-70An and WH-SFR-70An stock viruses. At 48 hpi they were stained with crystal violet.

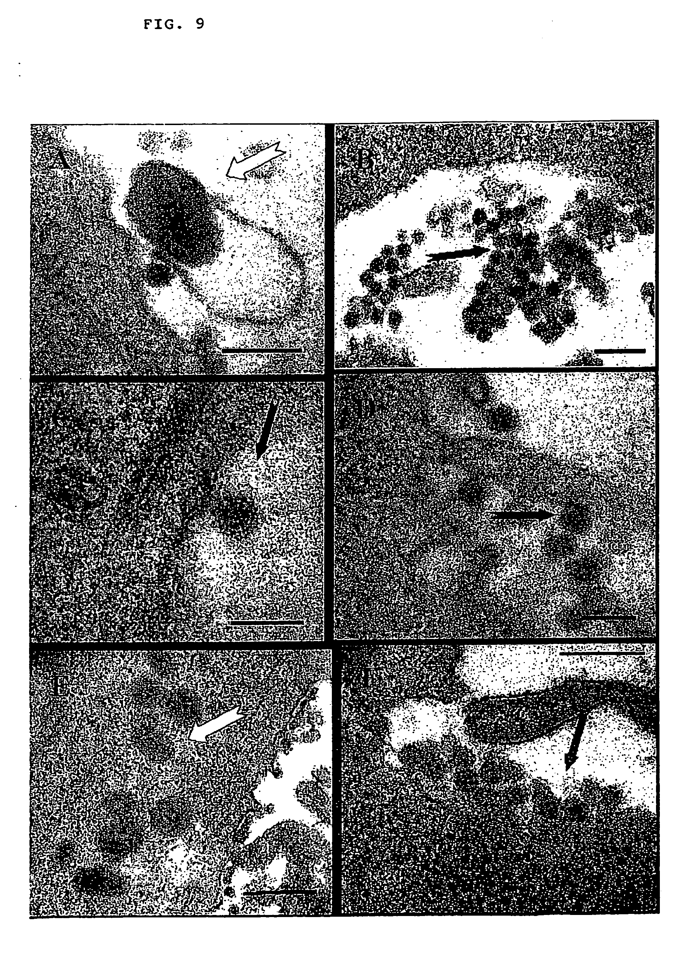

Figure 9 shows the images obtained through an electronic microscope of the WH-SFR 70An recombinant virus. The white arrows show vaccinia viruses and the black arrows point to SFPs (SFP, a similar particle to SFV that carries a packaged replicon, also referred to in this description as defective or single-cycle chimeric particle). Panel A (60K magnification) shows an enveloped extracellular vaccinia virus (EEV) with SFPs around it. In panels B (30K), C (60K), D (60K) and F (30K) SPFs appear in the cell membrane. Panel E (30K) shows a cell with intracellular vaccinia virus and SFPs outside the cell. A, C and D, bar 100 nm. B and F, bar 200 nm. E, bar 500 nm.

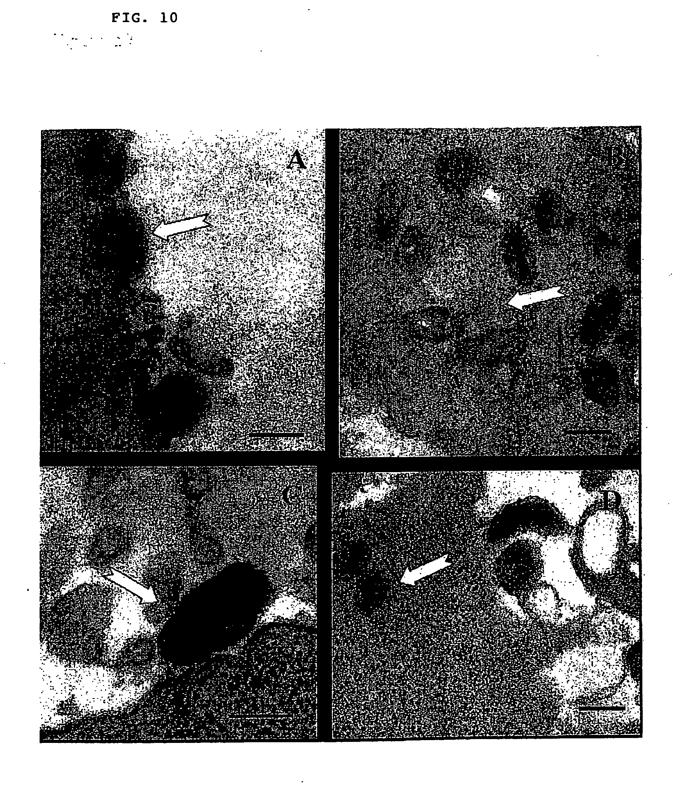

Figure 10 shows the images obtained through an electronic microscope of W-SFR 70An and WR. Panels A and B show cells infected with the W-SFR 70 recombinant virus. The arrows point to the vaccinia virus. A (30K magnification)-Cell-associated vaccinia virus (CEV). B (25K)-Shows intracellular enveloped vaccinia viruses (IEV). Panels C and D are cells infected with the vaccinia virus WR control. C (40K)-Cell-associated vaccinia virus (CEV). D (25K)-Intracellular enveloped virus (IEV). Note in all these cases, the absence of structures of the SFP type. Bar 200 nm.

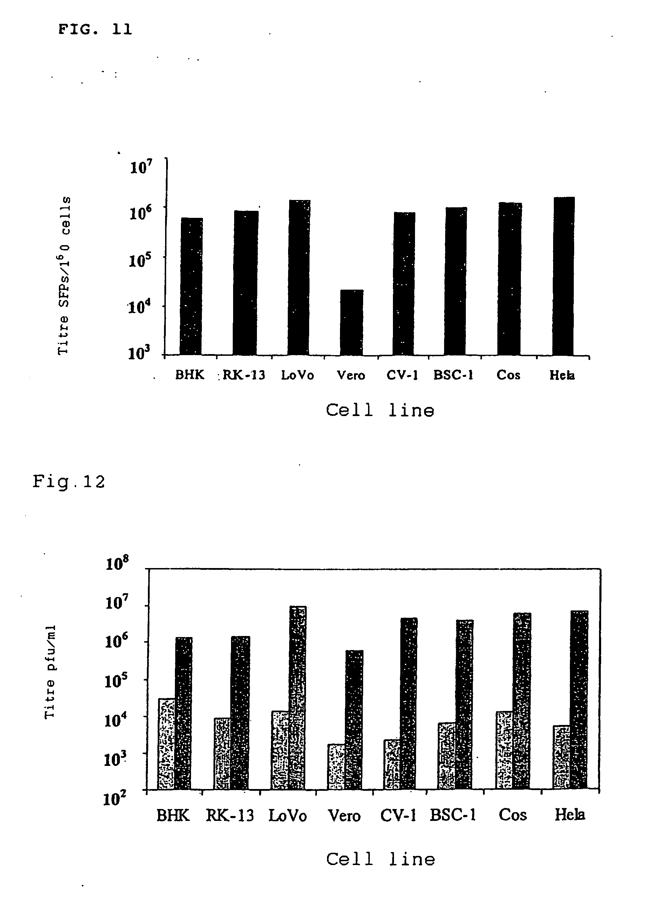

Figure 11 is a bar chart showing the production of SFPs in different cell lines. The cell lines being studied were infected with the WH-SFR 70An recombinant virus at an MOI of 5 pfu/cell. At 24 hpi the supernatants of the infections were harvested, cleared and filtered using a 0.1 µm filter. BHK cells seeded on 6-well plates were infected with the filtered medium and cells expressing GFP were counted at 24 hpi.
Figure 12 is a bar chart showing the production of extracellular vaccinia virus and cell-associated vaccinia virus from the WH-SFR 70An virus in different cell lines. The chosen cell lines were infected at a high MOI with the WH-SFR 70An virus. At 24 hpi the supernatants of the infections were harvested and cleared, separating supernatant (extracellular viruses) and cells (intracellular and cell-associated viruses). Plating was carried out on BSC-1 cells to evaluate the titre of the virus production. At 48 hpi it was stained with crystal violet. Light bars: Extracellular viruses. Dark bars: Cell-associated viruses.

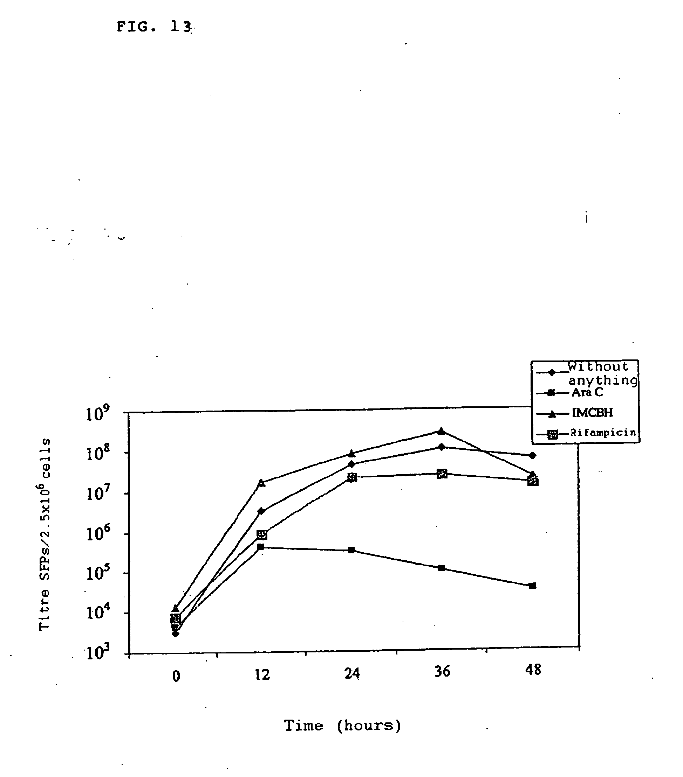

Figure 13 is a graph showing the kinetics of particle production (SFPs) in the presence of inhibitors of the vaccinia virus. Hela cells were infected at a high MOI with the WH-SFR 70 AN recombinant virus and after adsorption AraC (100 pg/ml) Rifampicin (100 pg/ml) or IMCBH (10 pg/ml) was added. Samples were taken every 12 hours, they were cleared, filtered and kept at 4°C until the last point in the kinetics was harvested. To determine the titre of SFPs, BHK cells were infected with 1:10 dilutions of the filtered supernatant and at 24 hpi GFP(+) cells were counted on the fluorescence microscope.

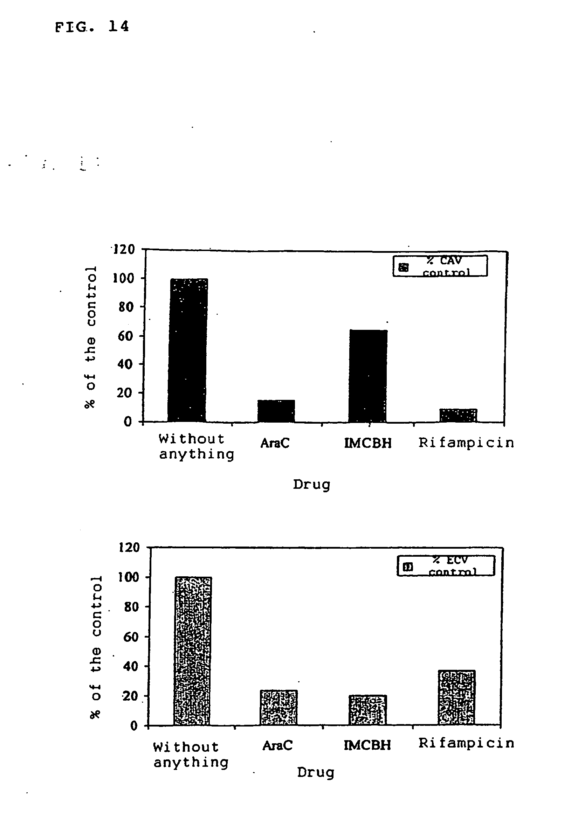

Figure 14 includes two graphs showing the production of extracellular and cell-associated viruses of the WH-SFR 70An virus. Hela cells were infected with the WH-SFR 70 An virus at an MOI of 5 pfu/cell and after 1 hour of adsorption AraC (100 pg/ml) Rifampicin (100 pg/ml)- or IMCBH (10 pg/ml) was added. The infections were harvested at 24 hpi and the titre of the virus production of extracellular and cell-associated viruses was evaluated. The production of extracellular viruses (ECV) and cell-associated viruses (CAV) was shown in relation to a control without an inhibitor.

### DETAILED DESCRIPTION OF THE INVENTION

An aspect of the invention relates to a recombinant DNA virus, referred to from here on as recombinant DNA virus of the invention, which comprises
(i) all or part of the genome of said DNA virus;
(ii) the complementary DNA sequence (cDNA) of a replicon deriving from an RNA virus
   wherein said replicon encodes its own replicase and contains a sequence of nucleotides that encodes an exogenous protein or fragment thereof and
   wherein said cDNA sequence is under the control of a transcription promoter of said DNA virus and
(iii) a nucleotide sequence that encodes structural proteins or encapsidating proteins of said RNA virus that package and transmit the replicon,
   wherein said nucleotide sequence is under the control of another transcription promoter of said DNA virus.

In a particular embodiment, the recombinant DNA virus of the invention comprises the whole genome of a DNA virus. In another particular embodiment, the recombinant DNA virus of the invention comprises part of the genome of a DNA virus; advantageously, in this case, the part of the genome of the DNA virus will include the elements needed for the replication of the DNA virus or, at the least, the elements needed to initiate the infection and transcription of the replicon. In another particular embodiment, the recombinant DNA virus of the invention comprises a part of the genome of the DNA virus and additional exogenous genes that can act as adjuvants or immunomodulators. By way of an example, deficient viruses could be used in non-essential functions or even single-cycle (abortive) DNA viruses affecting a single essential function in order to increase the safety of the system.

The term "replicon" as used in this description includes, although is not limited to, self-replicating single-strand positive-sense RNA molecules. Therefore, a replicon is a genetic element that encodes its own replicase and contains the necessary elements for its replication. The replicon, once inserted into a cell, is translated and then replicated. In the present invention, the replicon also includes a sequence of nucleotides that encode an exogenous protein or fragment thereof. The subgenomic promoter contained in the replicon regulates the transcription of the nucleotide sequence that encodes the exogenous protein or fragment thereof, as is shown in the diagram in Figure 5.

The replicons can be constructed by deleting all or part of the nucleotide sequences that encode the RNA virus capsid and by maintaining all the encoding nucleotide sequences that are needed for replication. The retention of genomic replication sequences makes it possible for the products of viral genes and exogens to be expressed in appropriate cells. Additional information about the construction of replicons can be found, for example, in WO 02/095023.

In general, the cDNA sequence of the replicon of the RNA virus used in the generation of the recombinant DNA virus of the invention comprises the nucleotide sequence that encodes the replicase of said RNA virus, the nucleotide sequence of a subgenomic promoter followed by the nucleotide sequence that encodes an exogenous protein or fragment thereof, the regions of the genome of said RNA virus that are needed for the replication of RNA and the RNA encapsidating signals. In a particular embodiment, said cDNA sequence comprises, in the 5' to 3' direction, (i) a 5' sequence that is required for amplification mediated by the RNA polymerase of the virus (replicase), (ii) a nucleotide sequence that encodes the replicase of said RNA virus, (iii) a means of expressing a nucleotide sequence that encodes an exogenous protein, (iv) a nucleotide sequence that encodes an exogenous protein or fragment thereof, and (v) a 3' sequence required for amplification mediated by the RNA polymerase of the virus (replicase), wherein said nucleotide sequence that encodes an exogenous protein replaces one or more genes that encode structural proteins of said RNA virus.

The cDNA sequence of the replicon deriving from the RNA virus that contains the nucleotide sequence that encodes an exogenous protein, or fragment thereof, is under the control of a transcription promoter of the DNA virus used in the generation of the recombinant DNA virus of the invention. Practically any promoter of said virus can be used. In a particular embodiment, the DNA virus is the vaccinia virus and the transcription promoter used for controlling the transcription of said cDNA sequence of the replicon deriving from the RNA virus is an early promoter of the vaccinia virus.

The exogenous protein, or fragment thereof, referred to in the recombinant DNA virus of the invention can be any protein, or fragment thereof, of interest, for example, in basic or applied research into human or animal health, etc., which is not natively present in the RNA virus or in the DNA virus used in the generation of the recombinant DNA virus of the invention. By way of an example, said exogenous protein can be chosen from a biologically active protein, a reporter protein, a cytotoxic protein, a protein of a pathogen, an immunomodulating protein, a protein of a tumour, etc. For example, the exogenous protein could be the gp120 glycoprotein of the human immunodeficiency virus (HIV) that causes AIDS, or the E2 glycoprotein of the Classic Swine Fever virus, the surface antigen of the Hepatitis B virus (HBV), the thymidine kinase of the Herpes Simplex virus, which makes it possible to kill selectively in the area of expression by using a drug, etc. In a particular embodiment, said exogenous protein is a reporter protein such as green fluorescent protein (GFP). Illustrative examples of fragments of exogenous proteins include antigens or epitopes of exogenous proteins, which can be used to immunise humans or animals.

The recombinant DNA virus provided by this invention also comprises a nucleotide sequence that encodes the structural proteins of the RNA virus used in the generation of the recombinant DNA virus of the invention or encapsidating proteins of said RNA. As used in this description, the term "encapsidating proteins" includes any protein with an encapsidating activity that leads to the transmission of the replicon to additional cells, that is, any protein that encapsidates (or packages) and transmits the replicon. In this way, both the replicon and structural or encapsidating proteins are produced in the cells infected by the recombinant DNA virus of the invention, which leads to the production of defective single-cycle chimeric virus particles that package the replicon, which can leave the cell and infect other cells. The nucleotide sequence that encodes the structural or encapsidating proteins of said RNA virus is located in a different locus of the genome of the DNA virus to the locus containing the cDNA sequence of the replicon deriving from the RNA virus that contains a nucleotide sequence that encodes an exogenous protein or a fragment thereof. In the event of said RNA virus having more than one structural protein, the encoding sequences of said structural proteins can be positioned adjacent to one another or separated, jointly controlled under the same promoter or by independent promoters. If only some of the structural or encapsidating proteins are necessary for the encapsidation and transmission of the replicon, only the corresponding genes will be included, positioned adjacent to one another or separated and jointly controlled under the same promoter or by independent promoters.

In a particular embodiment, the recombinant DNA virus provided by this invention comprises a nucleotide sequence that encodes the structural proteins of the RNA virus used in the generation of the recombinant DNA virus of the invention. In another particular embodiment, the recombinant DNA virus provided by this invention comprises a nucleotide sequence that encodes encapsidating proteins of the RNA virus used in the generation of the recombinant DNA virus of the invention.

The nucleotide sequence that encodes the structural or encapsidating proteins of the RNA virus used in the generation of the recombinant DNA virus of the invention is under the control of a transcription promoter of the DNA virus used in the construction of the recombinant DNA virus of the invention. Practically any promoter of said virus can be used. In one particular embodiment, the DNA virus is the vaccinia virus and the transcription promoter used to control the transcription of said nucleotide sequence that encodes the structural proteins of the RNA virus is an early/late promoter of the vaccinia virus. The promoter that regulates the transcription of the nucleotide sequence that encodes the structural proteins of the RNA virus is a promoter other than that which regulates the transcription of the cDNA sequence of the replicon deriving from the RNA virus that contains the nucleotide sequence that encodes an exogenous protein or fragment thereof. In a particular embodiment, the DNA virus is a vaccinia virus, the promoter used for controlling the transcription of the nucleotide sequence that encodes the structural proteins of the RNA virus is an early/late promoter of the vaccinia virus.

Practically any DNA virus can be used in the generation of the recombinant DNA virus of the invention; however, in a particular embodiment, said DNA virus is a double-strand DNA virus, for example, un poxvirus. In one particular embodiment, said DNA virus is a vaccinia virus (VV), a very large DNA virus (approximately 190 kb). Alternatively, other DNA viruses, such as herpesviruses or adenoviruses, could be used.

The RNA virus used in the generation of the recombinant DNA virus of the invention can be practically any virus with RNA genome; however, in a particular embodiment, said RNA virus is selected from the group consisting of the single-strand negative-sense RNA viruses, RNA viruses with segmented genome, double-strand RNA viruses and retroviruses (however, in the latter case the situation is more complicated as retroviruses are different in that the RNA is not self-replicating since it is generated by transcription when it is integrated into the cell DNA). In a particular embodiment, said RNA virus is an alphavirus, such as the Semliki Forest virus (SFV).

The recombinant DNA virus of the invention can be obtained by means of a process that consists of (i) obtaining the cDNA deriving from an RNA virus; (ii) constructing a replicon from said cDNA; (iii) inserting into said replicon a nucleotide sequence that encodes an exogenous protein or fragment thereof; (iv) inserting said cDNA into a locus of the genome of the DNA virus; and, if appropriate, (v) obtaining nucleotide sequences that encode the structural or encapsidating proteins of the RNA virus, and (vi) inserting said encoding nucleotide sequences of the structural or encapsidating proteins of the RNA virus into another locus of the genome of the DNA virus. The techniques used are conventional Genetic Engineering techniques that are well known by specialists in this subject (Sambrook et al., 1989).

In a particular embodiment (see the Example that accompanies this description) the construction of a VV-SFV recombinant DNA virus is described, which comprises the genome of the vaccinia virus (VV) wherein the following have been inserted into different loci: (i) a cDNA sequence of a replicon deriving from the Semliki Forest virus (SFV) that contains a nucleotide sequence that encodes the reporter protein GFP and (ii) a nucleotide sequence that encodes the structural proteins of the SFV particle. The objective of this particular embodiment was to combine two types of vectors, one based on DNA (VV) and the other based on RNA (SFV), in order to generate a vector that would act as a shuttle for the second (SFV). The expression vectors based on alphaviruses have been generated by substituting the genes that encode for the structural proteins of the virus for a heterologic gene, with the highly active promoter of subgenomic RNA maintaining transcriptional control (Berglund et al., 1993), (Liljestrom & Garoff, 1991). The RNA of the replicon can be transcribed in vitro and used directly by transfection or packaging in particles that are infectious by co-transfection in cell culture with a defective RNA Helper molecule, which provides the structural virion proteins in trans (Liljestrom & Garoff, 1991; Bredenbeek et al., 1993). In order to achieve the transcription of the SFV replicon, a recombinant vaccinia virus has been constructed that carries in its genome a complete replicon inserted into the locus of the thymidine kinase (TK) under the control of the natural early TK promoter. Additionally, a recombinant vaccinia virus was generated that also expressed the genes of the SFV structural proteins in the locus of the F13L gene under an early/late viral promoter. In the development of the combined vector VV-SFV, different aspects have been studied that are important for the viability of the system. Firstly, prior experiments had shown the compatibility of VV infections and SFV. The requirements regarding the promoter for transcription of the replicon have also been studied, as well as the requirements regarding the 3' end of the replicon (see the Example that accompanies this description).

The recombinant DNA virus of the invention also comprises a nucleotide sequence that encodes the structural proteins of the RNA virus used in the construction of the recombinant DNA virus of the invention or encapsidating proteins of said RNA virus. In this embodiment, when the recombinant DNA virus of the invention infects competent cells, it is in said cells that both the replicon and exogenous protein and the structural or encapsidating proteins of the RNA virus are produced. As a consequence of this, said structural or encapsidating proteins package the replicon (which also contains the encoding sequence of the exogenous protein or fragment thereof) to produce defective single-cycle chimeric virus particles that can leave the cell and infect other cells. Said virus particles therefore derive from the RNA virus and are chimeric because they contain a nucleotide sequence that encodes an exogenous protein or a fragment thereof, defective because the exogenous nucleotide sequence replaces all or part of the nucleotide sequences that encode the proteins of the RNA virus capsid, and single-cycle because they can only infect cells once as the packaged replicon lacks the nucleotide sequences that encode the capsid proteins of the RNA virus since they only contain the nucleotide sequences needed for replication and the nucleotide sequence that encodes the exogenous protein or fragment thereof.

Therefore, another aspect of the invention relates to a process for the production of defective single-cycle chimeric virus particles deriving from an RNA virus, which involves infecting competent cells with recombinant DNA viruses of the invention. After infection of the competent cells (that is, cells that are susceptible to be infected by the recombinant DNA virus of the invention), both the replicon and the structural or encapsidating proteins are produced, which leads to the production of defective single-cycle chimeric virus particles that package the replicon, which can leave the cell and are able to infect new cells. If desired, said defective single-cycle chimeric virus particles that are produced can be removed from the supernatant by conventional methods. In one particular embodiment, the production of defective chimeric SFV virus particles from a recombinant VV-SFV DNA virus is described (see the Example that accompanies this description).

The recombinant DNA virus of the invention presents numerous potential uses. In a particular embodiment, said recombinant DNA virus of the invention can be used as a system to generate packaged replicons. The advantage of this approach over the classic methods used to obtain packaged replicons is the fact that the in vitro transcription and electroporation of RNA would not be necessary, making the process more easily scalable. Also, the low or non-existent formation of viable RNA viruses is a considerable advantage.

In another particular embodiment, the recombinant DNA virus of the invention can be used to express exogenous proteins or fragments thereof in competent animal or plant cell systems through their infection with said recombinant DNA virus of the invention. Practically any exogenous protein, or fragment thereof, can be a protein, or fragment thereof, with a therapeutic action, for example, anti-tumour that acts on a malignant cell infected with said virus, or on a nearby cell, which works as a form of anti-tumour gene therapy. Therefore, in this case, the recombinant DNA virus of the invention together with an acceptable pharmaceutical vehicle can form a pharmaceutical composition. Non-limiting illustrative examples of acceptable pharmaceutical vehicles include water, oils, including mineral and vegetable oils, saline solutions, alcohol solutions, etc.

In another particular embodiment, said recombinant DNA virus of the invention can be used to provoke an immune response to the exogenous protein, or fragment thereof, in animals such as mammals, including humans. In this case, the recombinant DNA virus of the invention together with the acceptable pharmaceutical vehicle can form a recombinant vaccine. Non-limiting illustrative examples of acceptable pharmaceutical vehicles include water, oils, including mineral and vegetable oils, saline solutions, alcohol solutions, immunological adjuvants, etc. In a particular case, the replicon can express antigenic determinants of any pathogen, including bacteria, fungi, viruses or parasites. Alternatively, the replicon can express tumour antigens or epitopes deriving from antigens. Similarly, the replicon can express any biologically active protein, or fragment thereof, for example, an immunomodulating protein such as a cytokine, which can modulate the immune response in animals.

According to this application of the recombinant DNA viruses of the invention, it is possible to immunise animals against diseases through their direct use as recombinant vaccines. The recombinant DNA viruses of the invention are more effective than the singular vectors separately, as they can cause an amplification in the antigen expression, and because of their presentation to the immune system in two different cell contexts (cells infected with the recombinant DNA virus and cells infected with the defective single-cycle chimeric virus particle). It is well known that in immunisation using the vaccinia virus the host's response is directed against the proteins of the virus plus the antigen that is intended to be expressed. Occasionally, first a vaccine with DNA is administered in order to provoke a primary response to the protein of interest, and then a vaccine with the recombinant virus to generate a secondary response to said protein that is used as an antigen. By using the expression procedure of defective chimeric RNA virus particles produced from recombinant DNA. viruses of the invention, the host's immune response to a single protein is different in that the exogenous protein is the only protein expressed on a mass scale in both cells infected with the recombinant DNA virus of the invention and cells infected by defective single-cycle chimeric virus particles. The replicon system forms particles that increase the expression of the antigen, thus being spread more quickly and effectively in the host, centralising the host's response to a single protein. In a particular embodiment, the recombinant DNA virus of the invention comprises a nucleotide sequence that encodes encapsidating proteins with a certain specificity that can direct the single-cycle defective chimeric particles to certain types of cell.

Therefore, another aspect of the invention provides a pharmaceutical composition that comprises recombinant DNA viruses of the invention and an acceptable pharmaceutical vehicle. Said pharmaceutical composition includes the aforementioned recombinant vaccines.

The defective single-cycle chimeric virus particles provided by this invention are an additional object of the present invention and can be used for the same purposes as the recombinant DNA viruses of the invention.

Another aspect of the invention relates to a method for generating an immune response in animals such as mammals, including humans, that involves administering a recombinant vaccine to the animal that comprises recombinant DNA viruses of the invention in which the exogenous protein, or fragment thereof, is an immunogen.

Another aspect of the invention relates to a method for producing a protein of interest, or fragment thereof, which involves infecting a competent cell with recombinant DNA viruses of the invention. Once the protein, or fragment thereof, has been produced it can be recovered, if desired, either from the infected cell or from the medium (should it be excreted), using convention methods.

The following example illustrates how combined vectors of DNA (VV) and RNA (SFV) viruses are obtained and it must not be taken to limit the scope of this procedure. It is well known that vectors deriving from alphaviruses, such as Sindbis and Semliki Forest, are widely used for in vitro gene expression studies and are starting to be used to develop vaccines and for applications in gene therapy. The alphavirus family offers several advantages such as its wide host range (including mammal, insect and bird cells), high levels of protein expression and a relatively small genome that is easy to handle.

### EXAMPLE

Construction of the combined Vaccinia Virus-Semliki Forest Virus vector

This example was carried out in order to achieve combined vectors of DNA (VV) and RNA. (SFV) viruses. The design of these vectors is based on the transcription of a self-replicating RNA (replicon) and an RNA messenger of the DNA virus (VV). Since the replicon is translated in its replicase, once transcribed and translated, the replicon is independent from the gene expression machinery of the VV. In order to carry out this example, two different versions of combined vectors have been designed:
(1) Recombinant vaccinia viruses that express the complete SFV replicon, including an exogenous gene; and
(2) Recombinant vaccinia viruses that express the complete SFV replicon, including an exogenous gene, and that also express the proteins needed for packaging the replicon and so that may leave the cell, enter the extracellular medium and enter a new cell (Figure 5).

### I. MATERIALS AND METHODS

### I.1 Generating and isolating recombinant viruses

The recombinant viruses constructed for expression of the SFV replicon were isolated by infection/transfection in CV-1 cells (Obtained from the American Type Culture Collection, ATCC). In brief, cells seeded in a 9 cm² well at 80% confluence were infected with vRB12 vaccinia virus, a deletion mutant of the F13L gene in the WR strain (Blasco and Moss, 1992) at a multiplicity of infection (MOI) of 0.05 pfu/cell (plaque forming units per cell). After 1 hour of adsorption in a small volume of medium at 2% of serum they were transfected with Fugene (Sigma), as a transfection method and 2µg of plasmid. After 72 hours, the cells were harvested and lysed by three cycles of freezing and thawing. The virus obtained was diluted by 1:10 to infect monolayers of BSC-1 cells (obtained from the ATCC). At 48 hours post infection (hpi), plaques of the same size as the vaccinia virus, WR strain (Western Reserve), were isolated and resuspended in EMEM + 5% FCS (cell culture medium (EMEM), supplemented with 5% Foetal Calf Serum (FCS), both obtained from Biowiattaker). The plaque isolation procedure was repeated 3 more times, after which the double recombinant virus was amplified in BSC-1 cells.

The isolation of intermediary viruses for generation of the replicon was performed by a standard plating of vaccinia virus in 145B TK cells (obtained from the ATCC) in the presence of 25 µg/ml of bromodeoxyuridine (BrdU) (Calbiochem) and by monitoring the dsRed marker gene on the fluorescence microscope. The viruses expressing the replicon were isolated by means of green fluorescent protein (GFP) expression using a fluorescence microscope.

### I.2 Obtaining vaccinia virus containing the SFV replicon

### I.2.1 Construction of the pRed TS plasmid

Using DNA of cells infected with the WR virus and the pair of oligonucleotides identified as SEQ. ID. No. 1 and SEQ. ID. No. 2 as a template, a PCR product was generated that introduced a mutation into the natural thymidine kinase promoter of the vaccinia virus. This fragment was cloned in the Sph-I and Xho-I sites of the pGPTK plasmid (a plasmid generated in the inventors' laboratory) that had recombination flanks with the thymidine kinase gene resulting in the pGPTK* plasmid.

Two successive clonings were performed to construct a plasmid that would have recombination flanks with the genes of non-structural SFV proteins. The fragment f (3' end of the SFV replicon) was amplified with the pair of oligonucleotides identified as SEQ. ID. No. 3 and SEQ. ID. No. 4, and it was cloned in the pGPTK plasmid linearised with the BamHI enzyme. The resulting plasmid, pGPTKf, was used to clone part of the nsP1 gene (the 5' end of the SFV replicon) amplified by PCR with the oligonucleotides identified as SEQ. ID. No. 5 and SEQ. ID. No. 6. The amplified fragment was cloned in the Xho-I site of the pGPTK*f plasmid and the pGPTS plasmid was generated.

The fluorescent red protein (dsRed) was used as a marker gene for the selection of the recombinant viruses resulting from the transfection of this plasmid. The dsRed marker gene was obtained by digestion of the pRBdsRed2 plasmid (a plasmid generated in the inventors' laboratory) using Xho-I and BamHI enzymes and it was cloned in the pGPTS that had previously been digested using the same enzymes, thus removing the GFP-Pac cassette and inserting the dsRed gene. The plasmid created was named pRedTS-0 (Figure 1).

### I.2.2 Obtaining plasmids with mutations in the TK promoter, pRedTS

### 1-3

Mutations were introduced into the thymidine kinase promoter by PCR amplification of the TK_{L} and ns-1 fragments that overlap with the area of the promoter. Using the oligonucleotides identified as SEQ. ID. No. 7 and SEQ. ID. No. 8 and SEQ. ID. No. 9 (each of which introduces a mutation to the promoter that alters the intensity of expression) and SEQ. ID. No. 10, fragments ns-11, ns-12 and ns-13 that carry the mutations in the promoter and recombination flank ns-1 were generated. Using the oligonucleotides identified as SEQ. ID. No. 1 and SEQ. ID. No. 11, SEQ. ID. No. 12 and SEQ. ID. No. 13 (they introduce the same mutations to the promoter but in the complementary strand), fragments TK_{L} 1, TK_{L} 2 and TK_{L} 3 are amplified. The PCR products that overlap with the area of the promoter are used as a template to make recombinant PCR and thus amplify the fragments that carry the three versions of the promoter. The fragments with the three versions of the promoter are digested using Sph-I and Xho-I and they are cloned in the pRedTS plasmid that is digested using the same enzymes. Three plasmids were obtained: pRedTS 1, pRedTS 2 and pRedTS 3 (Figure 2). These plasmids were transfected in cells infected with the WR and V-Helper viruses and the W-RedTS 0, W-RedTS 1, W-RedTS 2 and W-RedTS 3 and WH-RedTS 0, WH-RedTS 1, WH-RedTS 2 and WH-RedTS 3 viruses were generated. These recombinant viruses are intermediaries for the formation of the vaccinia virus that expresses the SFV replicon. Using the pSFVrsGFP plasmid, to which a mutation was introduced in the gene sequence of the non-structural proteins to remove the termination signal of the transcription of vaccinia, and which has rsGFP cloned in the replicon, it was transfected in cells infected with the intermediary viruses. The resulting W-SFR 0, W-SFR 1, W-SFR 2 and W-SFR 3 and WH-SFR 0, WH-SFR 1, WH-SFR 2 and WH-SFR 3 viruses were isolated by GFP expression.

### I.2.3 Construction of the pRednsTK plasmid

A series of clonings were designed for the construction of the pRednsTK plasmid that would generate the intermediary recombinant viruses that are necessary for isolating the vaccinia virus that expresses the SFV replicon by means of infection/transfection in cells infected with the WR and V-Helper viruses. This strategy was used in order to study the requirement for 3' of the replicon.

Using the pRedTS 1 plasmid and by digestion using BamHI and Nsi-I, the right flank of the TK and the fragment f were removed and the right flank obtained by digestion using the same enzymes was inserted again. This plasmid was named pRedTK and the genes of the SFV non-structural proteins extracted from pSFV-1 (Liljestrom and Garoff 1991) by digestion using the same restriction endonucleases were inserted into it by digestion using BsiWI and BanHI and the fragment ns-1 and the dsRed marker that was cloned in a later step were removed. By PCR, with the pair of oligonucleotides identified as SEQ. ID. No. 14 and SEQ. ID. No. 15, the vaccinia promoter and the dsRed gene were amplified using the BamHI and BglII targets at their ends and it was cloned in the pnsTK plasmid digested using BamHI. This plasmid, pRednsTK, was transfected in cells infected with the WR and V-Helper viruses in order to generate the recombinant viruses W-RednsTK and WH-RednsTK (Figure 3). These recombinant viruses were created as intermediaries for the formation of the vaccinia virus that expresses the replicon. The pRednsTK plasmid was deposited in the Spanish Type Culture Collection (CECT) in Burjassot, Valencia, on 2 July 2003, under CECT access number 5824.

### I.2.4 Construction of the pMixf plasmid

Finally, the gene sequence of the SFV non-structural proteins, the vaccinia promoter, the dsRed marker and the left flank of the TK were removed using pnsRedTK and digestion with Sph-I and HindIII and the final part of the nsP4 gene and the rsGFP marker gene amplified by PCR using pSFV-rsGFP with the oligonucleotides identified as SEQ. ID. No. 16 and SEQ. ID. No. 17 were cloned. The plasmid created, pMix, had the restriction sites ZmaI and Stu-I to insert the sequence in 3' of the SFV replicon. Six fragments were amplified by PCR: F340, F340 poly A, F170, F170 poly A, F70 and F70 poly A (named according to the size and presence or not of poly A in the sequence).

**Table 1**

| Oligonucleotides used for amplification of the 3' end of the SFV replicon | |
|---|---|
| Pair of oligonucleotides | Amplified product |
| SEQ. ID. No. 18 - SEQ. ID. No. 19 | F de340 nt |
| SEQ. ID. No. 20 - SEQ. ID. No. 19 | F de170 nt |
| SEQ. ID. No. 21 - SEQ. ID. No. 19 | F de70 nt |
| SEQ. ID. No. 18 - SEQ. ID. No. 22 | F de340 nt + poly A |
| SEQ. ID. No. 20 - SEQ. ID. No. 22 | F de170 nt+ poly A |
| SEQ. ID. No. 21 - SEQ. ID. No. 22 | F de70 nt+ poly A |

These six fragments amplified by PCR had at their ends the Xmal and Stu-I restriction targets that were designed for their subsequent insertion in the pMix plasmid (Figure 4). The plasmids generated were transfected in cells infected with the W-Red nsTK and WH-Red nsTK viruses, thus creating the vaccinia virus that expresses the replicon. The pRednsTK plasmid was was deposited in the Spanish Type Culture Collection (CECT) in Burjassot, Valencia, on 2 July 2003, under CECT access number 5825. Also, the pRB-Helper plasmid, which contains the encoding sequence of the SFV structural proteins, was deposited in the Spanish Type Culture Collection (CECT) in Burjassot, Valencia, on 2 July 2003, under CECT access number 5823.

### II. RESULTS

### II.1 Study of the vaccinia virus promoter for the expression of the SFV replicon

Given that the characteristics of early messengers are more suitable for the transcription of larger RNAs (Davison and Moss 1989a, Davison and Moss 1989b), it was decided that the cDNA of the replicon (SFV) should be put under the control of the viral (VV) thymidine kinase (TK) promoter which is exclusively early (Weir and Moss 1987).

In order to test different possibilities with regard to transcription efficiency, it .was decided that different mutant TK promoters with different efficiency should be tested (Davison and Moss 1989a, Davison and Moss 1989b). The optimum sequence of the promoter and mutations that were introduced are described in Table 2.

**Table 2**

| Sequence of the mutated thymidine kinase promoters | | | |
|---|---|---|---|
| PROMOTER NAME | SEQ. ID. No. | SEQUENCE | RELATIVE EXPRESSION |
| 0 | 23 | 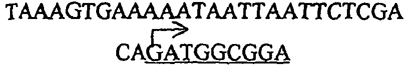 TAAAGTGAAAAATAATTAATTCTCGA CAGATGGCGGA | 189 |
| 1 | 24 | TAAAGTGAACAATAATTAATTCTCGA CAGATGGCGGA | 17 |
| 2 | 25 | TAAAGCGAACAATAATTAATTCTCGA CAGATGGCGGA | 5 |
| 3 | 26 | TAAAGTCAACAATAATTAATTCTCGA CAGATGGCGGA | Almost nil |

The mutated nucleotide in the sequence of the TK promoter is marked in bold and italics. The relative expression described for the different mutated promoters is specified (Davison and Moss 1989a, Davison and Moss 1989b). The underlined sequences correspond to the replicon. The initiation position for transcription is positioned just before the first nucleotide of the replicon (G) [see the arrow].

The construction of recombinant vaccinia viruses with mutated promoters and the SFV replicon was performed using a two-step process. Firstly, a visible marker (dsRed) was inserted into the TK locus together with the sequence of the 5' and 3' ends of the replicon. The replicon sequence was then completed by substituting the dsRed gene and inclusion of the genes of the non-structural proteins and the exogenous gene. This process was carried out using both the WR vaccinia virus and using the V-Helper virus (Lorenzo, Doctoral Thesis, 2001), which contains the genes of the SFV structural proteins. The viruses deriving from WR were named W-RedTS (intermediary vaccinia virus) and W-SFR and those deriving from V-Helper were named WH-RedTS (intermediary virus) and WH-SFR (Figure 6).

During the isolation process of the eight recombinant viruses, the GFP expression was monitored and the stability of the recombinant virus, which is an indicator of the viability or non-viability of the double recombinant. The results are shown in Table 3.

**Table 3**

| Recombinant viruses with different promoters | | | |
|---|---|---|---|
| VIRUS | PROMOTER SEQUENCE | STABLE RECOMBINANT | GFP EXPRESSION |
| W-SFR 0 | SEQ. ID. No. 27 | YES | + |
| W-SFR 1 | SEQ. ID. No. 28 | YES | + |
| W-SFR 2 | SEQ. ID. No. 29 | YES | + |
| W-SFR 3 | SEQ. ID. No. 30 | YES | + |
| WH-SFR 0 | SEQ. ID. No. 31 | NO | NA |
| WH-SFR 1 | SEQ. ID. No. 32 | YES | + |
| WH-SFR 2 | SEQ. ID. No. 33 | NO | NA |
| WH-SFR 3 | SEQ. ID. No. 34 | NO | NA |

On the left of the table the name of the virus is given with the mutated sequence of the TK promoter. The "Stable recombinant" column refers to whether it was possible to isolate the double recombinant virus. The GFP expression was viewed under the fluorescence microscope observing virus plaques. +: Indicates GFP expression. N: Not applicable.

The results suggest that, whilst in the W-SFR viruses the efficiency of the promoter does not affect the viability of the virus, it does in the WH-SFR viruses, which indicates that the SFV structural proteins have a role in the gene replication and/or expression of the replicon.

In order to test whether the isolated viruses were efficient in the formation of SFV pseudoparticles (SFPs), the presence of packaged replicons was determined in the supernatant of cultures infected by said viruses (Table 4). To do this, BHK-21 cells (obtained from the ATCC) were infected with the recombinant viruses W-SFR or WH-SFR. In parallel to this, coinfections of said recombinants were performed with the V-Helper virus, which provides "in trans" the proteins necessary for packaging the replicon for the W-SFR viruses that do not express structural proteins. The supernatants of the infections were harvested at 48 hpi and were filtered through a 0.1 µm filter to remove the vaccinia virus. Due to the size of the vaccinia virus (270 nm x 350 nm(Moss 1990b)) it is retained in the filter, whilst the SFPs pass through the filter. The presence of SFPs in the filtered medium was detected by infecting BHK-21 cells and observing the infections under the fluorescence microscope at 24 hpi in order to see cells with GFP expression.

**Table 4**

| Formation of single-cycle particles (SFPs) | | |
|---|---|---|
| RECOMBINANT VIRUS | GFP EXPRESSION | |
| | - V-HELPER | + V-HELPER (SFPs/ml) |
| W-SFR 0 | ≤ 2 | 4 X 10⁵ |
| W-SFR 1 | ≤ 2 | 6.4 X 10⁵ |
| W-SFR 2 | ≤ 2 | ≤ 2 |
| W-SFR 3 | ≤ 2 | ≤ 2 |
| WH-SFR 1 | 1.1 X 10⁶ | 9.6 X 10⁶ |

BHK-21 cells were infected with recombinant viruses or coinfected with recombinant viruses and the V-Helper virus. At 48 hpi the supernatant of the infection was cleared and filtered through 0.1 µm filters to remove the vaccinia virus particles. BHK-21 cells were infected with 500 µl and 50 µl of the supernatants and they were observed at 24 hpi under the fluorescence microscope in order to detect GFP expression. ≤ 2 indicates that no cell was detected with GFP fluorescence in the culture.

The results obtained (Tables 3 and 4) show that the strongest version of the promoter (version 0) gave the result that it is impossible to isolate the corresponding recombinant. Therefore, the weaker versions (versions 2 and 3) resulted in a low GFP expression or in a low production of particles. Therefore, on the basis of viability, GFP expression and the capacity to produce particles, version number 1 of the promoter was chosen as the most suitable for subsequent experiments.

### II.2 Effect of the sequence of the 3' end of the replicon

In order to determine the minimum requirement for the sequence of the 3' end of the SFV replicon with regard to expression of the exogenous gene and formation of SFPs, a number of recombinant viruses were constructed containing different versions of the SFV replicon with variations in the sequence of the 3' end thereof.

Firstly, recombinant vaccinia viruses are created that contain (in the TK locus) the gene sequence of non-structural SFV proteins under version 1 of the TK promoter and the dsRed marker gene. The W-RednsTK and WH-RednsTK viruses from the WR or V-Helper viruses, respectively, were isolated. These viruses are intermediaries for isolating the recombinant virus with the complete replicon.

The next step was to incorporate six fragments of different sizes (340, 170 and 70 nucleotides either including the PolyA sequence or not) corresponding to the 3' end area of the replicon that would serve to study the minimum requirement of the 3' end of the replicon. These 6 versions were inserted into both the virus deriving from WR and the virus deriving from V-Helper. The W-SFR 340, W-SFR 170 and W-SFR 70 or WH-SFR 340, WH-SFR 170 and WH-SFR 70 viruses (Figure 7) were thus isolated that contained in their genome the replicon with or without the PolyA sequence deriving from the cDNA of the replicon.

After the infections-transfections with the plasmids without PolyA no cells with green fluorescence were detected, it therefore being deduced that in said constructions there was no expression mediated by the replicon. On plating the progeny of these infected cultures, most of the virus plaques showed red fluorescence corresponding to the parental virus (Figure 7) and a small percentage of dsRed (-) plaques that corresponded to recombinant virus. These plaques did not show any green fluorescence. Therefore, the presence of PolyA sequence at the 3' end of the replicon is necessary in order for the exogenous gene to be expressed.

**Table 5**

| Study of the sequence of the 3' end of the replicon Recombinant Replicon cloned in the GFP expression | | | |
|---|---|---|---|
| virus | TK locus | W-SFR | WH-SFR |
| **W-SFR 340 WH-SFR 340** | 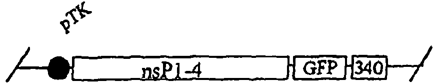 | - | - |
| **W-SFR 340 An WH-SFR 340 An** | 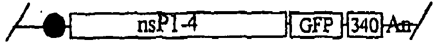 | + | + |
| **W-SFR 170 WH-SFR 170** | 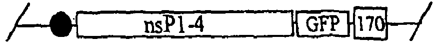 | - | - |
| **W-SFR 170 An WH-SFR 170 An** | 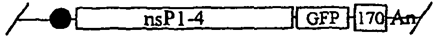 | + | + |
| **W-SFR 70 WH-SFR 70** | 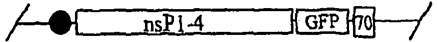 | - | - |
| **W-SFR 70 An WH-SFR 70 An** | 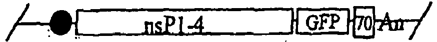 | + | + |

The diagram is shown for the constructions made in vaccinia viruses that contain the replicon with different sizes of 3' end of the replicon and with or without PolyA (An). GFP expression was visualised by observing the virus recombinant plaques under the fluorescence microscope. +: Indicates the presence of plaques that express GFP. -: Indicates that the plaques corresponding to the recombinant viruses were GFP (-).

The viruses that carried the SFV PolyA sequence and different sizes of the sequence of the 3' end of the replicon grew at high titres, comparable to those of the parental virus. The version with the sequence of 70 nucleotides at the 3' end was the most easily isolated in both the WH-SFR virus (containing the genes of the structural proteins) and the W-SFR virus. The isolation of the W-SFR and WH-SFR viruses with the other two versions, with 340 and 170 nucleotides, required more rounds of purification to isolate the double recombinant viruses.

These results show that 70 nucleotides at the 3' end of the replicon are sufficient and that the PolyA sequence is necessary for the correct expression of the exogenous gene mediated by the replicon. Therefore, all subsequent results refer to the versions that contain 70 nucleotides in the 3' position and the polyA sequence of the replicon.

### II.2 SFPs

### II.2.1 Formation of SFPs using the W-SFR and WH-SFR viruses

In order to check whether the W-SFR and WH-SFR constructions, with the three versions of the 3' end of the replicon, were able to package the replicon to form single-phase particles (SFPs), infections were performed using the recombinant viruses or coinfections using the V-Helper virus in BHK cells and the production of SFPs in the supernatant of the cultures was checked after 24 hours.

**Table 6**

| Production of SFPs using recombinant viruses | | |
|---|---|---|
| RECOMBINANT VIRUS | PRODUCTION OF SFPs (SFPs/ml) | |
| | -V-Helper | +V-Helper |
| W-SFR 340 | ≤2 | 2.9 X 10⁵ |
| W-SFR 170 | ≤2 | 3.05 X 10⁵ |
| W-SFR 70 | ≤2 | 2.1 X 10⁵ |
| WH-SFR 340 | 1.3 X 10⁵ | 2.3 X 10⁵ |
| WH-SFR 70 | 3.7 X 10⁵ | 4 X 10⁵ |

BHK-21 cells were infected with the recombinant viruses specified on the left of Table 6 (including the poly A of the replicon) or were coinfected with the recombinant viruses and the V-Helper virus. At 48 hpi the infection medium was harvested, cleared and filtered through 0.1 µm filters. Monolayers of BHK-21 cells were infected using 500 µl or 50 µ of the filtered medium. At 24 hpi they were observed under the fluorescence microscope and the number of cells that expressed GFP were counted.

The results show that the presence of the replicon in the absence of structural proteins does not produce detectable amounts of SFPs. However, the coinfection of recombinants that transcribe the replicon with the V-Helper virus produced high titres (≥10⁵) of SFPs, showing that the structural proteins expressed by V-Helper are able to package the replicon. Similarly, the recombinant viruses WH-SFR 340 An and WH-SFR 70 An were able, without coinfection, to produce similar titres of SFPs. Once it had been checked that the two versions of the recombinant virus WH-SFR worked correctly and produced similar titres of SFPs, the W-SFR 70 and WH-SFR 70 viruses were chosen (with the 70 nucleotides and polyA sequence version) as the most suitable for their later characterisation.

### II.2.2 Plaque morphology of the W-SFR 70 and WH-SFR 70 viruses

In order to compare the plaque morphology of the two isolated recombinant vaccinia viruses, W-SFR 70 and WH-SFR 70, a plate of the stock of these recombinant viruses was performed in BSC-1 cells in liquid medium. As can be seen in Figure 8, the infection with the W-SFR 70 virus produced normal vaccinia virus plaques. However, the recombinant virus WH-SFR 70 produced comet-shaped plaques. This is presumably due to the production of particles (SFPs) that are released into the medium and infect the surrounding cells. In fact, the comet-shaped plaque phenotype is described for strains of the vaccinia virus that release large amounts of extracellular virus. On the other hand, the plaque morphology of the W-SFR 70 virus is congruent with the fact that it does not form particles.

An unexpected observation in platings of the stock of the WH-SFR 70 virus was the presence of a small proportion of round plaques that did not correspond with the comet-shaped plaque phenotype. On observing these plaques under the fluorescence microscope, it was verified that these plaques did not express GFP. Viruses isolated from these plaques and regrown kept the GFP (-) phenotype, which means that they might be mutant viruses that have lost the ability to express GFP through the SFV replicon.

### II.3 Electronic microscopy of the WH-SFR 70 and W-SFR 70 viruses

In order to check the presence and morphology of particles generated from the WH-SFR 70 vaccinia virus, Hela cells (obtained from the ATCC) infected with the WR (vaccinia virus control), W-SFR 70 (recombinant vaccinia virus that expresses the replicon) or WH-SFR 70 (recombinant virus that forms particles) viruses were examined by electronic microscopy.

In the three samples, the different morphogenetic forms were distinguished that are distinctive of infection with vaccinia, including immature viruses, intracellular viruses mature (IMVs), intracellular enveloped viruses (IEVs) and cell-associated enveloped viruses (CEVs) (Figures 9 and 10).

In cells infected with WH-SFR 70, there was an apparent presence, in or close to the plasma membrane, of round particles of a considerably smaller size (48 +/- 4) than that of the vaccinia virus (200 +/- 20) (Figure 9). These particles, which in many cases appeared forming accumulations, are indistinguishable in size and appearance from mature SFV virions (Strauss and Strauss 1994). Similarly, typical images of said particles budding in the plasma membrane were frequently observed. The type and form of the particles was identical in cells treated with IMCBH (N-isonicotinoyl-N-3-methyl-chlorobenzoylhydrazine).

These results, together with the detection of the activity of the replicon in the supernatants of the infected cultures and the plaque phenotype of the WH-SFR 70 virus, show that the replicon is being packaged in particles that are morphologically and functionally equivalent to the SFV virus.

### II.4 Production of SFPs and production of vaccinia virus from the WH-SFR 70 virus in different cell lines

In the expression system based on SFV, the BHK cell line is usually used, in which high levels of expression and production of particles are achieved. The vaccinia virus, however, is usually grown and titred in human or monkey cells.

In order to determine efficiency in the production of SFPs from the WH-SFR 70 virus in different cell lines and to quantify viral production, a study was carried out on the following cell lines: BSC-1, CV-1, Cos, Hela, LoVo, BHK, RK-13 and Vero (obtained from the ATCC). To check the formation of vaccinia virus and SFV pseudoparticles, after infection of said cell lines, the production of SFPs, extracellular vaccinia virus and cell-associated vaccinia virus was evaluated.

### II.4.1 Production of SFPs from the WH-SFR 70 virus in different cell lines

The production of SFPs from the WH-SFR 70 virus was quantified using dilutions of the cleared supernatant of the infection that were used to infect monolayers of BHK cells.

From the results of Figure 11, it can be deduced that there are not any great variations in the production of SFPs in the cell lines used, with the exception of the Vero cells in which a smaller titre of particles was produced. From the lines tested, the Hela cells had the greatest efficiency in the production of SFPs, meaning that it was chosen for subsequent characterisation tests of the WH-SFR 70 virus.

### II.4.2 Production of extracellular vaccinia virus and cell-associated virus from the WH-SFR 70 virus in different cell lines

In order to characterise the biological properties of the WH-SFR 70 virus, the production of vaccinia virus was determined in different cell lines. To do this, the aforementioned cell lines were infected with the recombinant virus WH-SFR 70 and at 24 hpi the production of extracellular virus and cell-associated virus was quantified.

As can be seen in Figure 12, the production of extracellular virus and cell-associated virus from the WH-SFR 70 virus was very similar in the different cell lines used and was comparable to that of other recombinant viruses. From the cell lines tested, there was a slight decrease in the viral production in the Vero cells.

Therefore, in general terms the production of vaccinia virus from the recombinant virus WH-SFR 70 is not affected by the cell line in which the infection is performed, the production being similar to that obtained with other recombinant viruses.

### II.1 Particle production kinetics in the presence of vaccinia virus inhibitors

One of the parameters that was studied in order to optimise the expression and production of particles using the WH-SFR 70 virus is the time post-infection at which the infected cells had to be harvested to achieve the highest titre of particles. Moreover, the use of drugs that affect the replication or morphogenesis of the vaccinia virus was evaluated (Table 7), which in principle should not affect the replicon or the packaging thereof, as they are specific DNA (Ara C) or vaccinia morphogenesis (Rifampicin or IMCBH) drugs.

**Table 7**

| Vaccinia virus inhibitors used in the test | | |
|---|---|---|
| DRUG | EFFECT | CONCENTRATION USED (µg/ml) |
| Ara C Rifampicin IMCBH | Inhibits replication (DNA synthesis) | 100 |
| | Inhibits formation of IMV and EEV | 100 |
| | Inhibits formation of EEV | 10 |

In order to determine the optimum time post-infection for the production of SFV particles from the WH-SFR 70 virus, an infection was performed with the virus and samples were taken every 12 hours up to a maximum of 48 hours.

In the infected culture without the inhibitor, an increase was observed in the titre of particles up to 36 hours post-infection when the maximum production is reached. IMCBH and Rifampicin do not affect the formation of SFPs by the WH-SFR 70 virus (Figure 13). The addition of Ara C should not, in principle, negatively affect the replication of the replicon, as Ara C is specific to DNA and does not affect the synthesis of RNA. Therefore, the effect observed probably derives from a reduction in the amount of structural proteins needed to package the replicon.

In the cultures treated with Ara C, a certain amount of particle production is observed in the first few hours after infection, which is congruent with the exclusively early expression of SFV structural proteins in the presence of Ara C.

In view of the results, the optimum time post-infection for the formation of SFPs from the WH-SFR 70 virus is between 24 and 36 hpi. The addition of IMCBH slightly increases the particle titre.

### II.6 Viral production in the presence of vaccinia virus inhibitors

The production of extracellular and cell-associated vaccinia virus from the recombinant virus WH-SFR 70 was studied in the presence of the vaccinia virus inhibitors Ara C, IMCBH (Payne and Kristenson 1979) and Rifampicin (Moss, Rosenblum et al. 1969). For this purpose, Hela cells were infected with the WH-SFR 70 virus and at 24 hpi the extracellular virus in the supernatant of the infection and the cell-associated virus were quantified.

As has been described above, Ara C and Rifampicin (Moss, Rosenblum et al. 1969) reduce the production of extracellular and intracellular vaccinia virus. The extracellular and cell-associated virus production of the WH-SFR 70 virus was comparable to that of other recombinant viruses and it fitted the expected pattern. IMCBH inhibits the formation of extracellular enveloped virus (Payne and Kristenson 1979), so the percentage of extracellular virus production of the WH-SFR 70 virus was reduced by 80% compared to the control without an inhibitor, whilst the production of IMV only decreased by 20% in the presence of the drug.

It can be concluded that the behaviour of the WH-SFR 70 virus in viral production in the presence of vaccinia virus inhibitors is comparable to that of other recombinant vaccinia viruses.

Bearing in mind the results of the SFP production (Figure 13) and vaccinia virus production (Figure 14) kinetics , it can be concluded that the most suitable conditions for SFP production using the WH-SFR 70 virus are 36 hpi and in the presence of IMCBH to reduce the amount of vaccinia virus in the medium. In these conditions it is possible to achieve repeatable titres of SFPs that are close to 10⁸ SFPs/ml.

### II.7 Formation of viable Semliki Forest viruses

An important issue regarding the use of SFV as an expression vector is the possibility of forming the complete Semliki Forest virus, due to an RNA recombination process. In the expression system based on SFV, the virus can be generated by recombination between the recombinant RNA transfected molecules (replicon) and the messenger that contains the genes of the structural proteins (Geigenmüller-Gnirke, Weiss et al. 1991), (Weiss and Schlesinger 1991). It is also described that in the Sindbis virus model recombination occurs between the defective transcripts and that it is due to an exchange of templates during RNA replication (Weiss and Schlesinger 1991).

It is estimated that in the method based on the transfection of RNAs transcribed in vitro, the frequency of infective viruses found in a stock of packaged particles is 10⁻⁶ to 10⁻⁴, which implies the existence of infective SFV viruses in any stock of particles prepared using the method described. Although this is not very significant for the result of an expression experiment, it does represent a potential biosafety risk and limits its in vivo applications.

Considering the different design of our particle production system, we believe it to be of interest to check whether viable Semliki Forest viruses are formed in the SFP formation system using the WH-SFR 70 vaccinia virus. To do this, BHK cells were infected with the stock of 10⁷ particles that express GFP. At 48 hpi the monolayers of infected cells was observed under the fluorescence microscope and cells that expressed GFP were detected. The supernatant of this infection was harvested, cleared and filtered through a 0.1 µm filter and was used to infect another culture of BHK cells. At the same time, the supernatant was titred in BHK cells in semisolid medium in order to distinguish the SFV plaques. No evidence was found of infection by the SFV virus in either of the two cases.

It can therefore be concluded that the frequency of generation of viable SFV viruses in our system is lower that with the system based on plasmid transfection.

### III. DISCUSSION

The objective of this example has been to combine two types of vectors, one based on DNA (vaccinia virus) and the other based on RNA (Semliki Forest virus), in order to generate a vector that acts as a shuttle for the second (SFV).

Expression vectors based on Alphaviruses have been generated by substituting the genes that encode for the structural proteins of the virus for a heterologic gene, with the highly active subgenomic RNA promoter maintaining transcriptional control (Berglund et al., 1993), (Liljestrom & Garoff 1991). The RNA of the replicon can be transcribed in vitro and used directly by transfection or packaging in infectious particles by cotransfection in cell culture with a defective RNA Helper molecule that provides, in trans, the structural proteins of the virion (Liljestrom and Garoff 1991), (Bredenbeek et al., 1993).

To achieve the transcription of the SFV replicon, a recombinant vaccinia virus has been constructed that carries in its genome a complete replicon inserted into the thymidine kinase (TK) locus under the control of the natural early TK promoter. Additionally, a recombinant vaccinia was generated that also expressed the genes of the SFV structural proteins in the locus of the F13L gene under an early/late viral promoter. This work is the first time that a system thus designed has been seen. All published work contains different approaches. For example, research has been described using the vaccinia virus as a hybrid vector to produce particles of a retroviral vector ion packaged cells (Holzer & Falkner, 2003).

In the development of the combined VV-SFV vector, various important aspects have been studied for the viability of the system. Firstly, prior experiments had shown the compatibility of vaccinia infections and the Semliki Forest virus. This research also studied the requirements regarding the promoter for the transcription of the replicon and the requirements regarding the 3' end of the replicon.

The first step was to study the promoter of the vaccinia virus under which the replicon was to be expressed. The construction of the recombinant viruses that expressed the replicon using four versions of the promoter, of varying strengths of expression, served to study the influence of the promoter on the isolation and correct functioning of these viruses. The results show that a very active transcription of the replicon resulted in a very low expression of the exogenous gene. Using an intermediate version of the promoter it was possible to isolate the recombinant vaccinia virus and it was verified that it was efficient in the expression of the exogenous protein and in the formation of SFV particles.

Another important aspect in the isolation and efficiency of these viruses is the sequence requirement at the 3' end of the replicon. The replicon contains in its sequence the replicase gene, the subgenomic promoter followed by the heterologic gene that is to be expressed and the 5' and 3' end regions of the genome required for RNA replication (Niesters & Strauss, 1990), (Kuhn et al., 1990). The constructions carried out using different sizes of the 3' end sequence with and without PolyA revealed the need for the PolyA sequence in the 3' end of the replicon, regardless of the amount of adjacent sequence included. It must be mentioned that the PolyA sequence included in the constructions derives from the complementary DNA deriving from SFV, and that in any case, the early messengers of vaccinia are polyadenylated in their 3' end. Therefore, the results suggest not only the need for the PolyA sequence requirement but also more accurately the need for a correctly-positioned PolyA.

As regards the sequence requirements in the 3' end, the results show that 70 nucleotides are sufficient for the replicon to function. Therefore, the viruses chosen for their subsequent characterisation were those containing version "1" of the promoter, and the replicon with a 70-nucleotide 3' untranslated sequence and a PolyA tail.

The set of results supports the idea that in the WH-SFR 70 combined vector system pseudoparticles are produced containing functional packaged replicons that are the same as those generated by the traditional SFV system. This statement is based on a number of different observations:
i) the presence in supernatants of cells infected by WH-SFR 70 of small particles (filterable through 100 nm filters) that infect and produce expression of the heterologic gene in new cells;
ii) the comet-shaped plaque phenotype of the WH-SFR 70 virus, which indicates the release of infective particles into the culture medium; and
iii) the observation of icosaedric symmetry particles, similar to the SFV virus, in cells infected by WH-SFR 70 when observed under the electronic microscope.

Not all these observations occur for the WH-SFR 70 virus, which shows the requirement of structural SFv proteins so that the replicons may be packaged.

An important observation is the appearance, in the stocks of W-SFR 70 and WH-SFR 70 viruses, of mutants that have lost the expression mediated by the replicon. This can derive from an intrinsic instability of the construction or from the fact that the replication of the replicon competes to a certain extent with the replication of vaccinia, to the detriment of the latter. In the latter case, it is probable, although it occurs very infrequently, that said mutants grow more rapidly and are therefore rapidly enriched as the virus stocks growth are grown. This is obviously a significant practical problem for using the system and must be the object of future studies.

A crucial aspect regarding the utility and safety of the expression system based on SFV is the possibility that a stray SFV virus particle may arise by recombination between the RNA molecules of the replicon and the RNA Helper. In the original system, based on RNA cotransfection, it is described that this type of RNA recombination events may occur, which results in the generation of a population of infective SFV virus (Geigenmüller-Gnirke et al., 1991), (Weiss & Schlesinger, 1991). As the expression system provided by this invention is radically different as regards its design, tests were performed to check whether infective SFV viruses were generated from the WH-SFR 70 virus. To do this, a monolayers of cells was infected with the 10⁷ SFV particle stock and after two rounds of amplification no viable SFV virus was detected. This result suggests that the VV-SFV system is an improvement on the classic cotransfection systems.

The combined VV-SFV vector can have several applications. Firstly, it could be used as a system for generating packaged replicons. The advantage of this approach over the classic method is that the in vitro transcription and electroporation of RNA would not be necessary, which would make the process easily scalable. The low or non-existent formation of viable SFV viruses is also a substantial advantage.

A second potential advantage, which is perhaps of greater interest, is the use of combined vectors of this type in immunisation against diseases, that is, its direct use as recombinant vaccines. Combined vectors could be more effective than Singular vectors alone, as they can cause an amplification in the antigen expression and would be presented to the immune system in two different cell contexts.

In immunisation with the vaccinia virus the host response is directed against the proteins of the virus plus the antigen that is to be expressed. Occasionally, first a vaccine with DNA is administered in order to provoke a primary response to the proteins of the virus, and then the recombinant virus is administered in order to generate a secondary response to the protein that is used as an antigen. By using the procedure for the expression of Alphavirus particles based on the vaccinia virus, the host's immune response is singularised to one single protein, as the heterologic protein is the only one that is expressed massively in both cells infected with the recombinant vaccinia and cells infected by SFV pseudoparticles. The replicon system forms particles that increase the expression of the antigen and it could be expected that it would spread more efficiently and more rapidly throughout the host, centralising the host's response to a single protein.

### BIBLIOGRAPHY

Berglund, P., Sjoberg, M., Garoff, H., Atkins, G. J., Sheahan, B. J. & Liljestrom, P. (1993). Semliki Forest virus expression system: production of conditionally infectious recombinant particles. Biotechnology (N Y) 11, 916-20.
Blasco y Moss, 1992. Role of cell-associated enveloped vaccinia virus in cell-to-cell virus spread. J. Virol. 66, 4170-4179.
Bredenbeek, P. J., Frolov, I., Rice, C. M. & Schlesinger, S. (1993). Sindbis virus expression vectors: packaging of RNA replicons by using defective helper RNAs. J. Virology 67, 6439-6446.
Bredenbeek, P. J. & Rice, C. M. (1992). Animal RNA virus expression systems. Semin. Virol. 3, 297-310.
Davison, A. J. & Moss, B. (1989a). Structure of vaccinia virus early promoters. J Mol Biol 210, 749-69.
Davison, A. J. & Moss, B. (1989b). Structure of vaccinia virus late promoters. J Mol Biol 210, 771-84.
DiCiommo, D. P. & Bremner, R. (1998). Rapid, high level protein production using DNA-based Semliki Forest virus vectors. J. Biol. Chem. 273, 18060-18066.
Geigenmüller-Gnirke, U., Weiss, B. G., Wright, R. & Schlesinger, S. (1991). Complementation between Sindbis viral RNAs produces infectious particles with a bipartite genome. Proc. Natl. Acad Sci. 88, 3253-3257.
Holzer, G. W. & Falkner, F. G. (2003). Poxviral/Retroviral chimeric vectors allow cytoplasmic production of transducing defective retroviral particles. Methods Mol. Med. 76, 565-578.
Holzer, G.W., Mayrhofer, J., Gritschenberger, W., Dorner, F., & Falkner, F.G. (1999). Poxviral/retroviral chimeric vectors allow cytoplasmic production of transducing defective retroviral particles Virology 253, 107-144.
Konetschny, C., Holzer, G.W., Urban, C., Hämmerle, T., Mayrhofer, J., & Falkner, F.G. (2003). Generation of transduction-competent retroviral vectors by infection with a single hybrid vaccinia virus. J. Virology 77(12), 7017-7025.
Kuhn, R. J., Hong, Z. & Strauss, J. H. (1990). Mutagenesis of the 3'nontranslated region of Sindbis virus RNA. J. Virology 64, 1465-1476.
Lemm, J. A., Rumenapf, T., Strauss, E. G., Strauss, J. H. & Rice, C. M. (1994). EMBO J. 13, 2925-2934.
Liljestrom, P. (1994). Alphaviruses expression systems. Curr. Opin. Biotechnol. 5, 495-500.
Liljestrom, P. & Garoff, H. (1991). A new generation of animal cell expression vectors based on the Semliki Forest virus replicon. Biotechnology (NY) 9, 1356-61.
Lorenzo, M. M. (2001). Estudio de la localización de las proteinas de la envuelta de la forma extracelular del virus vaccinia. Tesis Doctoral.
Moss, B. (1990a). Poxviridae and their replication. In Virology, 2 edn, pp. 2079-2111. Edited by B. N. Fields & D. M. Knipe. New York: Raven Press.
Moss, B. (1990b). Regulation of vaccinia virus transcription. Annu.Rev.Biochem. 59, 661-688.
Moss, B., Rosenblum, E., Katz, E. & Grimley, P. (1969). Rifampicin: a specific inhibitor of vaccinia virus assembly. Nature 224, 1280-1284.
Niesters, H. G. M. & Strauss, J. H. (1990). Mutagenesis of the conserved 51-nucleotide region of Sindbis virus. J. Virology 64, 1639-1647.
Payne, L. G. (1979). Identification of the vaccinia hemagglutinin polypeptide from a cell system yielding large amounts of extracellular enveloped virus. J. Virol. 31, 147-155.
Sambrook, J., E.F., F. & Maniatis, T. (1989). Molecular cloning: a laboratory manual. Second edition. Cold Spring Harbor. Laboratory Press*.*
Schlesinger, P. & Garoff, H. (1987). The Togaviridae and Flaviviridae. Plenum Press. New York.
Schlesinger, S. & Dubensky, T. W., Jr. (1999). Alphaviruses vectors for gene expression and vaccines. Curr. Opin. Biotechnol. 10, 434-439.
Strauss, J. H. & Strauss, E. G. (1994). The alphaviruses: gene expression, replication and evolution. Microbiol. rev. 58, 491-562.
Weir, J. P. & Moss, B. (1987). Determination of the promoter region of an early vaccinia virus gene encoding thymidine kinase. Virology. 158, 206-210.
Weiss, B. G. & Schlesinger, S. (1991). Recombinantion between Sindbis virus RNAs. Journal of Virology 65, 4017-4025.
Xiong, C., Levis, R., Shen, P., Schlesinger, S., Rice, C. M. & Huang, H. V. (1989). Sindbis virus: an efficient, broad host range vector for gene expression in animal cells. Science 243, 1188-1191.

## Claims

1. A recombinant DNA virus that comprises
(i) all or part of the genome of said DNA virus;
(ii) the complementary DNA sequence (cDNA) of a replicon deriving from an RNA virus
wherein said replicon encodes its own replicase and contains a nucleotide sequence that encodes an exogenous protein or fragment thereof and
wherein said cDNA sequence is under the control of a transcription promoter of said DNA virus and
(iii) a nucleotide sequence that encodes structural proteins or encapsidating proteins of said RNA virus that package and transmit the replicon,
wherein said nucleotide sequence is under the control of another transcription promoter of said DNA virus.

2. Recombinant virus according to claim 1, wherein said DNA virus is a double-strand DNA virus.

3. Recombinant virus according to claim 2, wherein said DNA virus is chosen from the poxvirus, herpesvirus and adenovirus types.

4. Recombinant virus according to claim 3, wherein said DNA virus is a vaccinia virus.

5. Recombinant virus according to claim 1, wherein said RNA virus is chosen from the group comprising single-strand positive-sense RNA viruses, single-strand negative-sense RNA viruses, RNA viruses with segmented genome and double-strand RNA viruses.

6. Recombinant virus according to claim 5, wherein said RNA virus is an alphavirus.

7. Recombinant virus according to claim 6, wherein said alphavirus is the Semliki Forest virus (SFV).

8. Recombinant virus according to claim4, wherein the complementary DNA sequence (cDNA) of a replicon deriving from an RNA virus is under the control of an early transcription promoter of vaccinia virus and the nucleotide sequence that encodes structural proteins of said RNA virus is under the control of an early/late transcription promoter of vaccinia virus.

9. Recombinant virus according to claim 8, wherein the early transcription promoter of vaccinia is a TK Promoter selected from SEQ ID No. 27, SEQ ID No. 28, SEQ. No. 29, SEQ ID No. 30 and SEQ ID No. 32.

10. Recombinant virus according to claims 6 or 7, that further comprises a polyA sequence at the 3' of the replicon.

11. Recombinant virus according to claim 10, wherein the polyA sequence has 70 nucleotides.

12. Recombinant virus according to claim 1, wherein said exogenous protein, or fragment thereof, is a biologically active protein, a reporter protein, a cytotoxic protein, a protein of a pathogen, an immunomodulating protein, a protein of a tumour, an antigen or an epitope of an antigen.

13. Recombinant virus according to claim 12, wherein said exogenous protein, or fragment thereof, is chosen from the gp120 glycoprotein of the human immunodeficiency virus (HIV) that causes AIDS, the E2 glycoprotein of the Classic Swine Fever virus, the surface antigen of the Hepatitis B virus (HBV) and the thymidine kinase of the Herpes Simplex virus.

14. A procedure for the production of defective single-cycle chimeric virus particles containing a replicon of an RNA virus that involves infecting competent cells with recombinant DNA viruses according to any of claims 1 to 13.

15. Procedure according to claim 14, which also involves removing the virus particles produced.

16. A pharmaceutical composition that comprises recombinant DNA viruses according to any of claims 1 to 13 and an acceptable pharmaceutical excipient.

17. A method for producing a protein of interest, or fragment thereof, which involves infecting a competent cell with recombinant DNA viruses according to any of claims 1 to 13, and, if desired, recovering said protein of interest or fragment thereof.

## Patentansprüche

1. Rekombinantes DNS-Virus umfassend
(i) die Gesamtheit oder einen Teil des Genoms des DNS-Virus;
(ii) die komplementäre DNS-Sequenz (cDNS) eines von einem RNS-Virus abgeleiteten Replikons
wobei das Replikon seine eigene Replikase kodiert und eine Nukleotidsequenz enthält, die ein exogenes Protein oder ein Fragment davon kodiert und
wobei die cDNS-Sequenz unter der Kontrolle eines Transkriptionspromotors des DNS-Virus steht und
(iii) eine Nukleotidsequenz, die strukturelle Proteine oder einhüllende Proteine des RNS-Virus kodiert, die das Replikon verpacken und übertragen,
wobei die Nukleotidsequenz unter der Kontrolle eines anderen Transkriptionspromotors des DNS-Virus steht.

2. Rekombinantes Virus gemäß Anspruch 1, wobei das DNS-Virus ein doppelsträngiges DNS-Virus ist.

3. Rekombinantes Virus gemäß Anspruch 2, wobei das DNS-Virus ausgewählt ist aus den Pockenvirus-, Herpesvirus- und Adenovirustypen.

4. Rekombinantes Virus gemäß Anspruch 3, wobei das DNS-Virus ein Vacciniavirus ist.

5. Rekombinantes Virus gemäß Anspruch 1, wobei das RNS-Virus ausgewählt ist aus der Gruppe umfassend einsträngige positiv strangorientierte RNS-Viren, einsträngige negativ strangorientierte RNS-Viren, RNS-Viren mit segmentiertem Genom und doppelsträngige RNS-Viren.

6. Rekombinantes Virus gemäß Anspruch 5, wobei das RNS-Virus ein Alphavirus ist.

7. Rekombinantes Virus gemäß Anspruch 6, wobei das Alphavirus das Semliki-Forest-Virus (SFV) ist.

8. Rekombinantes Virus gemäß Anspruch 4, wobei die komplementäre DNS-Sequenz (cDNS) eines von einem RNS-Virus abgeleiteten Replikons unter der Kontrolle eines frühen Transkriptionspromotors von Vacciniavirus steht und die Nukleotidsequenz, die strukturelle Proteine des RNS-Virus kodiert, unter der Kontrolle eines frühen/späten Transkriptionspromotors von Vacciniavirus steht.

9. Rekombinantes Virus gemäß Anspruch 8, wobei der frühe Transkriptionspromotors von Vaccinia ein TK-Promotor ist, der ausgewählt ist aus SEQ ID Nr. 27, SEQ ID Nr. 28, SEQ ID Nr. 29, SEQ ID Nr. 30 und SEQ ID Nr. 32.

10. Rekombinantes Virus gemäß Anspruch 6 oder 7, das zusätzlich eine polyA-Sequenz am 3'-Ende des Replikons enthält.

11. Rekombinantes Virus gemäß Anspruch 10, wobei die polyA-Sequenz 70 Nukleotide aufweist.

12. Rekombinantes Virus gemäß Anspruch 1, wobei das exogene Protein oder Fragment davon ein biologisch aktives Protein, ein Reporter-Protein, ein zytotoxisches Protein, ein Protein eines Krankheitserregers, ein immunmodulierendes Protein, ein Protein eines Tumors, ein Antigen oder ein Epitop eines Antigens ist.

13. Rekombinantes Virus gemäß Anspruch 12, wobei das exogene Protein oder Fragment davon ausgewählt ist aus dem gp120 Glykoprotein des Humanen Immundefizienz-Virus(HIV), welches AIDS verursacht, dem E2 Glykoprotein des klassischen Schweinepest-Virus, dem Oberflächen-Antigen des Hepatitis-B-Virus (HBV) und der Thymidinkinase des Herpes-simplex-Virus.

14. Verfahren zur Herstellung von defekten chimären Viruspartikeln mit einem einzigen Vermehrungszyklus, die ein Replikon eines RNS-Virus enthalten, welches die Infektion kompetenter Zellen mit rekombinanten DNS-Viren gemäß einem der Ansprüche 1 bis 13 umfasst.

15. Verfahren gemäß Anspruch 14, das auch die Entfernung der hergestellten Viruspartikeln umfasst.

16. Pharmazeutische Zusammensetzung, die rekombinante DNS-Viren gemäß einem der Ansprüche 1 bis 13 und einen verträglichen pharmazeutischen Hilfsstoff umfasst.

17. Verfahren zur Herstellung eines interessierenden Proteins oder eines Fragments davon, das die Infektion einer kompetenten Zelle mit rekombinanten DNS-Viren gemäß einem der Ansprüche 1 bis 13 umfasst, und, wenn dies gewünscht ist, die Gewinnung des interessierenden Proteins oder Fragments davon.

## Revendications

1. Virus à ADN recombinant qui comprend :
(i) tout ou partie du génome du dit virus à ADN,
(ii) la séquence d'ADN complémentaire (ADNc) d'un réplicon dérivant d'un virus à ARN,
où le dit réplicon code pour sa propre réplicase et contient une séquence nucléotidique qui code pour une protéine exogène ou un fragment de celle-ci et
où la dite séquence d'ADNc est sous le contrôle d'un promoteur de transcription du dit virus à ADN et
(iii) une séquence nucléotidique qui code pour des protéines structurelles ou des protéines d'encapsidation du dit virus à ARN qui emballent et transmettent le réplicon,
où la dite séquence nucléotidique est sous le contrôle d'un autre promoteur de transcription du dit virus à ADN.

2. Virus recombinant selon la revendication 1, **caractérisé en ce que** le dit virus à ADN est un virus à ADN double brin.

3. Virus recombinant selon la revendication 2, **caractérisé en ce que** le dit virus à ADN est choisi parmi les virus de type : pox-virus, herpès virus et adénovirus.

4. Virus recombinant selon la revendication 3, **caractérisé en ce que** le dit virus à ADN est un virus de la vaccine.

5. Virus recombinant selon la revendication 1, **caractérisé en ce que** le dit virus à ARN est choisi dans le groupe composé des virus à ARN simple brin de sens positif, les virus à ARN simple de sens négatif, les virus à ARN à génome segmenté et les virus à ARN double brin.

6. Virus recombinant selon la revendication 5, **caractérisé en ce que** le dit virus à ARN est un alphavirus.

7. Virus recombinant selon la revendication 6, **caractérisé en ce que** le dit alphavirus est le virus de la forêt de Semliki (VFS).

8. Virus recombinant selon la revendication 4, **caractérisé en ce que** la séquence d'ADN complémentaire (ADNc) d'un réplicon dérivant d'un virus à ARN est sous le contrôle d'un promoteur de transcription précoce du virus de la vaccine et la séquence nucléotidique qui code pour les protéines structurelles du dit virus à ARN est sous le contrôle d'un promoteur de transcription précoce/tardif du virus de la vaccine.

9. Virus recombinant selon la revendication 8, **caractérisé en ce que** le promoteur de transcription précoce de la vaccine est un promoteur TK choisi parmi les SEQ ID N° 27, SEQ ID N°28, SEQ ID N°29, SEQ ID N°30 ET SEQ ID N°32.

10. Virus recombinant selon la revendication 6 ou 7, comprenant en outre une séquence polyA à l'extrémité 3' du réplicon.

11. Virus recombinant selon la revendication 10, **caractérisé en ce que** la séquence polyA comporte 70 nucléotides.

12. Virus recombinant selon la revendication 1, **caractérisé en ce que** la dite protéine exogène, ou un fragment de celle-ci, est une protéine biologiquement active, une protéine rapporteuse, une protéine cytotoxique, une protéine d'un pathogène, une protéine immuno-modulatrice, une protéine d'une tumeur, un antigène ou un épitope d'antigène.

13. Virus recombinant selon la revendication 12, **caractérisé en ce que** la dite protéine exogène ou le fragment de celle-ci, est choisi parmi la glycoprotéine gp120 du virus de l'immunodéficience humaine (VIH) qui cause le SIDA, la glycoprotéine E2 du virus de la fièvre porcine classique, l'antigène de surface du virus de l'hépatite B (VHB) et la thymidine kinase du virus herpès simplex.

14. Procédé pour la production de particules virales chimériques défectives à cycle unique contenant un réplicon d'un virus à ARN qui implique l'infection de cellules adéquates avec des virus à ADN recombinant selon l'une quelconque des revendications 1 à 13.

15. Procédé selon la revendication 14, qui implique aussi l'extraction des particules produites.

16. Composition pharmaceutique qui comprend des virus à ADN recombinant selon l'une quelconque des revendications 1 à 13 et un excipient pharmaceutiquement acceptable.

17. Méthode de production d'une protéine intéressante, ou d'un fragment de celle-ci, qui implique d'infecter une cellule adéquate avec des virus à ADN recombinant selon l'une quelconque des revendications 1 à 13, et, si on le souhaite, de récupérer la dite protéine intéressante ou le fragment de cette dernière.


### LIST OF SEQUENCES

<110> National Institute for Agriculture and Food
   Research and Technology (INIA)
<120> Recombinant DNA viruses that comprise a
   complementary DNA of a replicon of an RNA virus and its
applications
<160 34
<170> PatentIn version 2.0
<210> 1
   <211> 28
   <212> DNA
   <213> Artificial sequence
<223> TK_{LL} oligonucleotide
<400> 1
   gcttttgcat gcaataaatg gatcacaa 28
<210> 2
   <211> 28
   <212> DNA
   <213> Artificial sequence
<223> TK_{LR*} oligonucleotide
<400> 2
   gacactcgag aattaattat ttttcact 28
<210> 3
   <211> 24
   <212> DNA
   <213> Artificial sequence
<223> SFV 11370 Bam oligonucleotide
<400> 3
   aattggatcc ttacataagc ttaa 24
<210> 4
   <211> 21
   <212> DNA
   <213> Artificial sequence
<223> SFV 11900 Bgl oligonucleotide
<400> 4
   cggacagatc tccggtaagc g 21
<210> 5
   <211> 25
   <212> DNA
   <213> Artificial sequence
<223> SFV 700 Xho oligonucleotide
<400> 5
   gacactcgag aattaattat ttttcact 25
<210> 6
   <211> 25
   <212> DNA
   <213> Artificial sequence
<223> ns-1 Sal oligonucleotide
<400> 6
   aggtgagtcg acagatggcg gatgt 25
<210> 7
   <211> 30
   <212> DNA
   <213> Artificial sequence
<223> pTK1f oligonucleotide
<400> 7
   gaataaagtg aacaataatt aattctcgac 30
<210> 8
   <211> 30
   <212> DNA
   <213> Artificial sequence
<223> pTK2f oligonucleotide
<400> 8
   gaataaagcg aacaataatt aattctcgac 30
<210> 9
   <211> 30
   <212> DNA
   <213> Artificial sequence
<223> pTK3f oligonucleotide
<400> 9
   gaataaagtc aacaataatt aattctgac 30
<210> 10
   <211> 25
   <212> DNA
   <213> Artificial sequence
<223> SFV 700 Xho oligonucleotide
<400> 10
   cacctgctcg agggccagt ttgtg 25
<210> 11
   <211> 29
   <212> DNA
   <213> Artificial sequence
<223> pTK1R oligonucleotide
<400> 11
   gaattaatta ttgttcactt tattcgact 29
<210> 12
   <211> 29
   <212> DNA
   <213> Artificial sequence
<223> pTK2R oligonucleotide
<400> 12
   gaattaatta ttgttcgctt tattcgact 29
<210> 13
   <211> 29
   <212> DNA
   <213> Artificial sequence
<223> pTK3R oligonucleotide
<400> 13
   gaattaatta ttgttgactt tattcgact 29
<210> 14
   <211> 26
   <212> DNA
   <213> Artificial sequence
<223> Red Bam 5' oligonucleotide
<400> 14
   gtcttaggat ccaactcgag aaaaat 26
<210> 15
   <211> 26
   <212> DNA
   <213> Artificial sequence
<223> Red Bgl 3' oligonucleotide
<400> 15
   tctccagatc tataaaaatt atttat 26
<210> 16
   <211> 24
   <212> DNA
   <213> Artificial sequence
<223> ns-4f oligonucleotide
<400> 16
   cggtcggcat gcaacgagat gtca 24
<210> 17
   <211> 26
   <212> DNA
   <213> Artificial sequence
<223> SFVGFP Hind oligonucleotide
<400> 17
   gggatgtaat aagcttaatt acccgg 26
<210> 18
   <211> 24
   <212> DNA
   <213> Artificial sequence
<223> F 340 Xma oligonucleotide
<400> 18
   gcagaaaatc ccgggtggtc tggg 24
<210> 19
   <211> 26
   <212> DNA
   <213> Artificial sequence
<223> F Stu R oligonucleotide
<400> 19
   tttggaaggc ctaaaaacca attgca 26
<210> 20
   <211> 25
   <212> DNA
   <213> Artificial sequence
<223> F 170 Xma oligonucleotide
<400> 20
   ctgtatccg ggtaacaaag cgcaa 25
<210> 21
   <211> 25
   <212> DNA
   <213> Artificial sequence
<223> F70 Xma oligonucleotide
<400> 21
   ggcaataccc gggagcttac ataag 25
<210> 22
   <211> 25
   <212> DNA
   <213> Artificial sequence
<223> F Stu R poly A oligonucleotide
<400> 22
   gcgcgtaggc ctattcatta atgca 25
<210> 23
   <211> 37
   <212> DNA
<223> Mutated thymidine kinase promoter of vaccinia virus
   "0"
<400> 23
   taaagtgaaa aataattaat tctcgacaga tggcgga 37
<210> 24
   <211> 37
   <212> DNA
<223> Mutated thymidine kinase promoter of vaccinia virus
   "1"
<400> 24
   taaagtgaac aataattaat tctcgacaga tggcgga 37
<210> 25
   <211> 37
   <212> DNA
<223> Mutated thymidine kinase promoter of vaccinia virus
   "2"
<400> 25
   taaagcgaac aataattaat tctcgacaga tggcgga 37
<210> 26
   <211> 37
   <212> DNA
<223> Mutated thymidine kinase promoter of vaccinia virus
   "3"
<400> 26
   taaagtcaac aataattaat tctcgacaga tggcgga 37
<210> 27
   <211> 25
   <212> DNA
<223> W-SFR 0 virus promoter
<400> 27
   taaagtgaaa aataa 15
<210> 28
   <211> 15
   <212> DNA
<223> W-SFR 1 virus promoter
<400> 28
   taaagtgaac aataa 15
<210> 29
   <211> 15
   <212> DNA
<223> W-SFR 2 virus promoter
<400> 29
   taaagcgaac aataa 15
<210> 30
   <211> 15
   <212> DNA
<223> W-SFR 3 virus promoter
<400> 30
   taaagtgaac aataa 15
<210> 31
   <211> 15
   <212> DNA
<223> WH-SFR 0 virus promoter
<400> 31
   taaagtgaaa aataa 15
<210> 32
   <211> 15
   <212> DNA
<223> WH-SFR 1 virus promoter
<400> 32
   taaagtgaac aataa 15
<210> 33
   <211> 15
   <212> DNA
<223> WH-SFR 2 virus promoter
<400> 33
   taaagcgaac aataa 15
<210> 34
   <211> 15
   <212> DNA
<223> WH-SFR 3 virus promoter
<400> 34
   taaagtcaac aataa 15